# FASCICULE DE BREVET EUROPEEN

(11) **EP 0 672 147 B1**
(45) Date de publication et mention de la délivrance du brevet: **26.03.2008**
(21) Numéro de dépôt: 94902830.2
(22) Date de dépôt: 17.12.1993
(51) Int. Cl.: C12N 15/31, C12Q 1/68, G01N 33/569, C12P 21/08, C12N 15/52

(54) **PROTEINE CONFERANT UNE RESISTANCE DE TYPE INDUCTIBLE A DES GLYCOPEPTIDES, NOTAMMENT CHEZ DES BACTERIES A GRAM-POSITIF**
PROTEIN DIE EINE INDUZIERBARE RESISTENZ GEGEN GLYCOPEPTIDE VERURSACHT , INSBESONDERE BEI GRAM-NEGATIVEN BAKTERIEN
PROTEIN CONFERRING AN INDUCIBLE RESISTANCE TO GLYCOPEPTIDES, PARTICULARLY IN GRAM-POSITIVE BACTERIA

(30) Priorité: 18.12.1992 FR 9215671; 07.07.1993 FR 9308356
(43) Date de publication de la demande: 20.09.1995
(73) Titulaire: INSTITUT PASTEUR, 75724 Paris Cédex 15 (FR)
(72) Inventeur: ARTHUR, Michel, F-75010 Paris (FR); DUTKA-MALEN, Sylvie, F-94260 Fresnes (FR); EVERS, Stefan, F-75013 Paris (FR); COURVALIN, Patrice, F-75015 Paris (FR)
(74) Mandataire: Desaix, Anne
(86) Numéro de dépôt international: PCT/FR1993/001264
(87) Numéro de publication internationale: WO 1994/014961

(56) Documents cités:
- WO-A-92/07942
- GENE vol. 112, no. 1 , 1 Mars 1992 , AMSTERDAM NL pages 53 - 58 DUTKA-MALEN, S. ET AL. 'Sequence of the vanC gene of Enterococcus gallinarum BM4174 encoding a D-alanine:D-alanine ligase-related protein necessary for vancomycin resistance' cité dans la demande
- FEMS MICROBIOLOGY LETTERS vol. 70, no. 1 , 15 Juin 1990 , AMSTERDAM, NL pages 101 - 106 AL-OBEID, S. ET AL. 'Comparison of vancomycin-inducible proteins from four strains of Enterococci' cité dans la demande
- ANTIMICROBIAL AGENTS AND CHEMOTHERAPY vol. 34, no. 10 , Octobre 1990 pages 1875 - 1879 DUTKA-MALEN, S. ET AL. 'Phenotypic and genotypic heterogeneity of glycopeptide resistance determinants in Gram-positive bacteria' cité dans la demande
- GENE vol. 124, no. 1 , 14 Février 1993 , AMSTERDAM NL pages 143 - 144 EVERS, D. ET AL. 'The vanB gene of vancomycin-resistant Enterococcus faecalis V583 is structurally related to genes encoding D-Ala:D-Ala ligases and glycopeptide-resistance proteins VanA and VanC'
- GENE vol. 140, no. 1 , 11 Mars 1994 , AMSTERDAM NL pages 97 - 102 EVERS, S. ET AL. 'Sequence of the vanB and ddl genes encoding D-alanine:D-lactate and D-alanine:D-alanine ligases in vancomycine-resistant Enterococcus faecalis V583'

## Description

L'invention concerne les polypeptides associés à l'expression d'une résistance à des antibiotiques de la famille des glycopeptides, cette résistance étant de type inductible par la vancomycine et non inductible par la teicoplanine, notamment chez des bactéries à Gram-positif, en particulier de la famille des cocci à Gram-positif. L'invention vise également une séquence nucléotidique codant pour ces polypeptides. Elle concerne aussi l'utilisation de ces polypeptides et de leur séquence nucléotidique en tant que moyens de détection in vitro d'une résistance à des glycopeptides. Parmi les cocci à Gram-positif, l'invention vise tout particulièrement les entérocoques, les streptocoques et les staphylocoques.

Les glycopeptides, parmi lesquels la vancomycine et la teicoplanine, sont des antibiotiques inhibiteurs de la synthèse de la paroi bactérienne. Ces antibiotiques sont très utilisés pour le traitement des infections sévères dues à des cocci à Gram-positif (entérocoques, streptocoques et staphylocoques), en particulier dans les cas d'allergie et de résistance aux pénicillines.

Jusqu'en 1986, la vancomycine s'est avérée efficace contre la quasi-totalité des souches d'entérocoques.

L'activité des glycopeptides dépend de la formation d'un complexe entre l'antibiotique et les précurseurs du peptidoglycane, plus que de l'interaction directe avec des enzymes du métabolisme de la paroi cellulaire. En particulier on a constaté que les glycopeptides se fixent aux résidus terminaux D-alanyl-D-alanine (D-ala-D-ala) des précurseurs du peptidoglycane.

Plusieurs phénotypes de résistance aux glycopeptides ont été mis en évidence ; on a notamment distingué des souches résistantes à un haut niveau de glycopeptides et les souches résistantes à un bas niveau de concentration.

Par souche résistante à un haut niveau, on entend une souche de bactérie en particulier une souche de cocci à Gram-positif pour laquelle les concentrations minimales inhibitrices (CMI) de vancomycine et de teicoplanine sont respectivement supérieures à 32 et 8 µg/ml. Les CMI de la vancomycine vis à vis des souches résistantes à bas niveau sont comprises entre 8 et 32 µg/ml. Le phénotype VanB est caractérisé par une résistance inductible par la vancomycine, mais non inductible par la teicoplanine. Une fois induite, cette résistance peut exister vis à vis de différents glycopeptides, en particulier vis à vis de la vancomycine et/ou de la teicoplanine, et ce à des niveaux variables.

Les souches d'entérocoques répondant au phénotype VanB (classe B) sont notamment des souches de E.faecalis et E.faecium.

Al-Obeid S. et al (FEMS Microbiology Letters 70 (1990) 101-106) ont ainsi comparé les protéines de résistance à des glycopeptides, inductibles par la vancomycine, chez quatre souches Enterococci, et ont déduit de leur comparaison, l'existence de trois types de protéines, l'un de ces types étant présent chez la souche E.faecium résistante à bas niveau de vancomycine. Selon les auteurs de cette publication, une protéine de poids moléculaire d'environ 39,5 kDa serait induite chez les souches à bas niveau de résistance et cette résistance serait liée à une induction par la vancomycine. Ces souches présenteraient aussi une résistance à la teicoplanine, également induite par la vancomycine.

D'après Al-Obeid et al, cette protéine de 39,5 kDa serait présente sous de multiples formes mais la nature de cette multiplicité n'a pas été étudiée. Selon ces auteurs, il pourrait exister une spécificité de structure en fonction des espèces concernées de bactéries et du niveau de résistance, ce qui demande à être confirmé.

Dans cette publication, Al-Obeid et al ont décrit 11 acides aminés de la séquence N-terminale de cette protéine de 39,5 kDa et ont observé que cette séquence comportait environ 70% d'homologie avec de nombreuses protéines de membrane d'origine procaryote ou eucaryote, ayant des fonctions diverses. Cette comparaison ne permettait pas selon les auteurs d'établir la fonction éventuelle de la protéine. Enfin Al-Obeid et al ont remarqué que d'autres protéines sont induites, à un degré moindre cependant.

L'invention concerne des peptides, polypeptides ou protéines impliqués dans l'expression d'une résistance à des antibiotiques de la famille des glycopeptides et notamment à la vancomycine et/ou à la teicoplanine, ainsi que les séquences nucléotidiques codant pour de tels polypeptides. La résistance dont il est question ci-dessus est de type inductible par la vancomycine et non par la teicoplanine.

Les expressions "impliqué dans l'expression d'une résistance" ou "impliqué dans une résistance" signifient que la protéine de l'invention est nécessaire pour que se manifeste la résistance.

L'invention vise également des sondes nucléotidiques utilisables pour la détection d'une résistance aux glycopeptides, notamment par la réaction de polymérisation en chaîne (PCR), ou par des tests faisant intervenir des anticorps.

L'invention a donc pour objet une protéine VanB caractérisée en ce qu'elle comprend la séquence d'acides aminés suivante I, et en ce qu'elle participe à la résistance à des glycopeptides, en particulier à la vancomycine, cette résistance étant de type inductible par la vancomycine et non par la teicoplanine, chez des bactéries à Gram-positif.

Par l'expression "résistance inductible" on entend la capacité pour une bactérie à Gram-positif déterminée, en particulier pour une souche Enterococcus déterminée, de produire une protéine VanB en présence d'une concentration 0,05 à 1 µl/ml de vancomycine.

La résistance à un ou plusieurs glycopeptides déterminés peut se traduire par la persistance d'une infection due à des germes habituellement sensibles aux glycopeptides, ou peut être détectée au moyen d'un antibiogramme (surtout pour les hauts niveaux de résistance), de la CMI, de l'hybridation avec des sondes (après amplification par exemple par PCR).

Selon un premier mode particulier de réalisation de l'invention, la protéine VanB est caractérisée en ce qu'elle est impliquée dans une résistance de type inductible à des glycopeptides et en particulier à la vancomycine, chez des entérocoques et par exemple chez des souches du genre E. faecium ou E. faecalis.

L'invention concerne également une protéine VanB caractérisée en ce qu'elle comprend une séquence d'acides aminés modifiée par rapport à la séquence I, par délétion, insertion, ou remplacement d'un ou plusieurs acides aminés, dès lors que la protéine VanB ainsi modifiée est impliquée chez des bactéries à Gram-positif, dans une résistance à des glycopeptides, en particulier à la vancomycine, cette résistance étant de type inductible par la vancomycine, et non inductible par la teicoplanine.

Entre également dans le cadre de l'invention, tout fragment peptidique de la protéine VanB caractérisé en ce qu'il correspond à la séquence d'acides aminés I ou à toute partie de cette séquence fonctionnellement associée à la résistance de type inductible à des glycopeptides, en particulier à la vancomycine, chez des bactéries à Gram-positif, par exemple des bactéries de la famille des enterocoques.

Avantageusement des fragments peptidiques de l'invention présentent en outre ou alternativement, des propriétés antigéniques et sont donc reconnus par des anticorps formés contre la protéine VanB.

Un fragment particulier de la séquence I correspond par exemple à la séquence suivante, ou comprend cette séquence :

Selon un autre mode de réalisation de l'invention, ces antigènes sont spécifiques de la protéine VanB et ne sont donc pas reconnus par des anticorps reconnaissant les protéines VanA et VanC telles que décrites dans la demande de brevet EP 91920753.

L'invention concerne en outre une séquence de nucléotides caractérisée en ce qu'elle code pour une protéine VanB impliquée chez des bactéries à Gram-positif, dans une résistance à des glycopeptides, en particulier à la vancomycine, cette résistance étant de type inductible par la vancomycine et non inductible par la teicoplanine, ladite protéine VanB comprenant la séquence d'acides aminés I, ou en ce qu'il s'agit d'une séquence d'ADN complémentaire de cette séquence codante, ou d'une séquence d'ARN correspondante.

Par séquence complémentaire, on entend toute séquence d'ADN dont les nucléotides sont complémentaires de ceux de la séquence I, et dont l'orientation est inversée.

Une séquence nucléotidique particulière répondant à cette définition est caractérisée en ce qu'elle comprend l'enchaînement de nucléotides II suivant ou un enchaînement nucléotidique modifié par rapport à II, dès lors qu'il code pour une protéine impliquée chez des bactéries à Gram-positif, dans une résistance à des glycopeptides, en particulier à la vancomycine, cette résistance étant de type inductible par la vancomycine et non inductible par la teicoplanine.

De manière générale l'invention a également pour objet un fragment nucléotidique caractérisé en ce qu'il est capable d'hybrider dans des conditions stringentes avec une séquence telle que définie dans l'enchaînement II ci-dessus.

Les conditions stringentes sont les suivantes :
* température de la réaction 65°C pendant une nuit dans une solution contenant 0,1% de SDS, 0,7% de lait en poudre écrémé, 6xSSC (1xSSC=0,15M NaCl et 0,015M de citrate de sodium à pH=7,0)
* lavages à température ambiante dans 2xSSC-0,1% SDS puis à 65°C dans 0,2SSC-0,1% SDS.

Avantageusement un fragment nucléotidique répondant à la définition précédente aura au moins 15 nucléotides, de préférence au moins 20.

Une séquence nucléotidique particulière comprend à cet égard l'enchaînement suivant:

Les peptides et polypeptides de l'invention permettent de définir une classe génotypique, caractérisée par la capacité des séquences nucléotidiques codant pour ces peptides, d'hybrider dans des conditions stringentes avec la séquence II constituant une sonde.

Ces fragments peuvent être mis en oeuvre en tant qu'amorces pour la réalisation de réactions d'amplification, ou en tant que sondes.

Des amorces particulièrement intéressantes répondent aux séquences suivantes :
- amorce 1 : 5' ATGGGAAGCCGATAGTC 3' (positions 241-258 des nucléotides de la séquence I)
- amorce 2 : 5' GATTTCGTTCCTCGACC 3' (séquence réverse-complémentaire du fragment des nucléotides 860 à 877 de la séquence I).

Des sondes nucléotidiques selon l'invention peuvent être spécifiques pour détecter chez des bactéries à Gram-positif des séquences codant pour une protéine VanB impliquée dans la résistance à des glycopeptides notamment à la vancomycine et/ou à la teicoplanine, cette résistance étant inductible conformément à la définition précédente, ces sondes pouvant en outre être universelles parmi ces séquences.

Par les sondes spécifiques de vanB, on entend tout oligonucléotide hybridant avec une séquence nucléotidique codant pour une protéine VanB selon l'invention, telle que décrite dans les pages précédentes, et ne présentant pas de réaction d'hybridation croisée ou d'amplification (PCR) avec des séquences présentes chez l'ensemble des souches sensibles.

Un fragment nucléotidique particulier selon l'invention est caractérisé en ce qu'il n'hybride pas dans des conditions stringentes avec l'ADN de souches d'entérocoques sensibles à la vancomycine, en particulier avec l'ADN des souches E. faecalis JH2-2 et E. faecium BM4107.

Ces souches de référence ont respectivement été décrites par Jacobs et Hobbs (J. Bacteriol. 117, 1974, 360-372) et Leclercq et al (Antimicrob. Agents Chemother 33, 1989).

Un autre fragment nucléotidique intéressant dans le cadre de l'invention, est spécifique du gène vanB, dans la mesure où il n'hybride pas dans des conditions stringentes avec les gènes vanA et vanC tels que décrits dans la demande PCT 91920753.

Un fragment nucléotidique particulièrement intéressant dans le cadre de l'invention, est le fragment répondant à la séquence II.

Ce fragment est un fragment interne issu du gène impliqué dans la résistance à des souches d'entérocoques. La résistance peut exister à des niveaux variables de concentration en glycopeptides.

L'invention concerne aussi des fragments de nucléotides modifiés par rapport aux précédents, par mutation, addition ou délétion de nucléotides, dès lors que le fragment ainsi modifié soit code pour un fragment de la protéine VanB fonctionnel s'agissant de son association à la résistance à des glycopeptides, en particulier à la vancomycine, dans les conditions décrites ci-dessus, soit hybride avec le gène vanB.

Dans l'hypothèse d'une utilisation des fragments nucléotidiques à titre de sondes, un marquage sera effectué, par les techniques classiques. A titre d'exemple, on utilisera des marqueurs radioactifs ou enzymatiques.

Des fragments de nucléotides selon l'invention peuvent être mis en oeuvre en tant qu'amorces, pour réaliser l'amplification, par exemple par PCR, d'acide nucléique contenu dans un échantillon biologique donné.

L'invention vise par ailleurs une séquence d'ADN recombinante, caractérisée en ce qu'elle comprend une séquence de nucléotides ci-dessus décrite, sous le contrôle d'éléments de régulation susceptibles d'intervenir dans le clonage ou l'expression, d'un gène impliqué dans une résistance de type inductible par la vancomycine et non inductible par la teicoplanine, à des antibiotiques de la famille des glycopeptides, notamment la vancomycine, chez un hôte déterminé.

Ce gène impliqué dans la résistance est par exemple le gène vanB qui comprend la séquence de nucléotides II ou toute partie fonctionnelle en terme de résistance inductible, issue d'une séquence hybridant avec la séquence II.

L'invention concerne aussi un vecteur recombinant pour le clonage ou l'expression, caractérisé en ce qu'il comprend une séquence nucléotidique ci-dessus décrite en un site non essentiel pour sa réplication, le cas échéant sous le contrôle d'éléments de régulation susceptibles d'intervenir dans l'expression d'une résistance de type inductible par la vancomycine et non inductible par la teicoplanine, à des antibiotiques de la famille des glycopeptides, notamment la vancomycine, chez un hôte déterminé.

Des vecteurs particuliers sont par exemple des plasmides, des phages, des cosmides, des YAC.

Un vecteur préféré est le plasmide pAT201, déposé à la C.N.C.M. le 11 Décembre 1992 sous le numéro I-1277.

Un autre vecteur préféré est le plasmide pAT202 formé à partir du plasmide pUC19Ω contenant un fragment de 3,3 kb contenant le gène vanB de Enterococcus faecalis V583 (HindIII/KpnI).

pAT202 a été introduit dans E. coli JM83 et déposé à la CNCM le 29 Mars 1993, sous le n° I-1291 (identification E. coli BM2973).

Ces vecteurs peuvent être utilisés pour transformer ou transfecter des hôtes cellulaires afin de cloner ou d'exprimer les séquences nucléotidiques de l'invention.

Un hôte cellulaire recombinant selon l'invention est caractérisé en ce qu'il est modifié par une séquence nucléotidique ou un vecteur ci-dessus décrits.

De préférence l'hôte cellulaire est modifié par cette séquence dans des conditions permettant l'expression d'une protéine VanB fonctionnelle s'agissant de la résistance inductible à des glycopeptides.

L'invention a aussi pour objet une protéine VanB recombinante telle qu'obtenue à partir d'un hôte cellulaire recombinant selon la définition précédente, la protéine VanB obtenue étant caractérisée en ce que son ossature peptidique comprend la séquence d'acides aminés ci-dessus, et en ce qu'elle est impliquée chez des bactéries à Gram-positif, dans une résistance à des glycopeptides, en particulier à la vancomycine, cette résistance étant de type inductible par la vancomycine et non inductible par la teicoplanine.

La protéine VanB selon l'invention permet de préparer des anticorps monoclonaux ou polyclonaux caractérisés en ce qu'ils reconnaissent spécifiquement la protéine VanB ou un fragment peptidique décrit ci-dessus.

Ces anticorps peuvent être obtenus selon les méthodes classiques de production d'anticorps. En particulier pour la préparation des anticorps monoclonaux on aura recours à la méthode de Kohler et Milstein selon laquelle on prépare des anticorps monoclonaux par fusion cellulaire entre des cellules de myélome et des cellules spléniques de souris préalablement immunisées avec un polypeptide ou une composition selon l'invention, conformément au procédé classique.

Les anticorps de l'invention sont avantageusement utilisables pour la détection de la présence de protéines caractéristiques d'une résistance aux glycopeptides, en particulier à la vancomycine et à la teicoplanine, cette résistance étant du type inductible par la vancomycine et non inductible par la teicoplanine.

Entre également dans le cadre de l'invention, un kit pour le diagnostic in vitro sur un échantillon biologique, de la présence de souches résistantes à des glycopeptides après induction notamment par la vancomycine et non par la teicoplanine, ces souches appartenant en particulier aux cocci à Gram-positif, notamment en ce qu'il s'agit des souches d'entérocoques, par exemple E. faecium, caractérisé en ce qu'il comprend :
- des anticorps ci-dessus décrits, le cas échéant marqués,
- un réactif pour la détection d'une réaction immunologique du type anticorps-antigène,
- le cas échéant des réactifs pour effectuer la lyse des cellules de l'échantillon testé,
- le cas échéant une concentration déterminée de vancomycine, pour induire la résistance.

L'invention concerne aussi un kit tel que défini ci-dessus, qui comprend en outre des anticorps dirigés spécifiquement contre la protéine VanA et/ou des anticorps dirigés spécifiquement contre la protéine VanC.

Selon un autre mode de réalisation de l'invention, le kit permet indifféremment la détection d'une résistance correspondant à un phénotype VanA, VanB ou VanC, et comprend des anticorps reconnaissant VanA, VanB et VanC. Ces anticorps peuvent être sélectionnés par leur capacité à reconnaître un épitope commun aux trois protéines. Il peut également s'agir d'un mélange d'anticorps reconnaissant des épitopes différents, propres à chacune des protéines.

Selon une autre mode de réalisation de l'invention, un kit pour le diagnostic in vitro de la présence de souches résistantes à bas niveau, à des glycopeptides, en particulier résistantes à la vancomycine, est caractérisé en ce qu'il comprend :
- une sonde nucléotidique capable d'hybrider dans des conditions stringentes avec une séquence nucléotidique du gène vanB, et le cas échéant,
- des nucléoside-triphosphates dATP, dCTP, dTTP, dGTP,
- un agent de polymérisation d'ADN.

Un autre kit de détection comprend en outre une sonde de nucléotides capable d'hybrider spécifiquement avec le gène vanA et une sonde capable d'hybrider spécifiquement avec le gène vanC.

Ce kit peut être avantageusement utilisé pour la détection d'une résistance chez des cocci à Gram-positif notamment chez des entérocoques, par exemple chez E. faecium.

L'invention vise aussi un kit pour la détection in vitro d'une résistance à des glycoprotéines, notamment à la vancomycine, cette résistance correspondant à l'un des phénotypes VanA, VanB ou VanC, le kit comprenant
- une sonde nucléotidique hybridant avec les gènes vanA, vanB et vanC,
- des nucléoside-triphosphates dATP, dCTP, dTTP et dGTP,
- un agent de polymérisation d'ADN.

L'invention vise aussi un procédé de détection in vitro de la présence de souches résistantes à des glycopeptides, en particulier à la vancomycine et/ou à la teicoplanine, ces souches appartenant en particulier à la famille des cocci à Gram-positif, notamment en ce qu'il s'agit de souches d'entérocoques, par exemple E. faecium ou E.faecalis, caractérisé en ce qu'il comprend :
a) la mise en contact d'un échantillon biologique susceptible de contenir les souches résistantes, avec une amorce constituée par un fragment nucléotidique selon l'invention, tel que décrit ci-dessus, capable d'hybrider avec une séquence nucléotidique recherchée, impliquée dans l'expression de la résistance, cette séquence étant utilisée comme matrice, en présence des 4 différents nucléosides triphosphates, et d'un agent de polymérisation, dans des conditions d'hybridation telles que pour chaque séquence nucléotidique ayant hybride avec une amorce, un produit d'élongation de chaque amorce complémentaire de la matrice est synthétisé,
b) la séparation de la matrice et du produit d'élongation obtenu, ce dernier pouvant alors également se comporter comme une matrice,
c) la répétition de l'étape a) de façon à obtenir une quantité détectable des séquences nucléotidiques recherchées,
d) la détection du produit d'amplification des séquences nucléotidiques.

La sonde utilisée peut donc être spécifique de la séquence nucléotidique II ou d'une séquence hybridant dans des conditions stringentes avec la séquence II. Dans ces conditions, le procédé selon l'invention permet la détection d'une résistance à des glycopeptides, cette résistance étant inductible par la vancomycine et non inductible par la teicoplanine.

Selon un mode de réalisation particulier de l'invention, ce procédé comprend également la mise en contact de l'échantillon biologique avec un fragment nucléotidique spécifique du gène vanA et/ou un fragment nucléotidique spécifique du gène vanC. Dans ce cas le procédé selon l'invention permet avantageusement la détection de différents phénotypes de résistance.

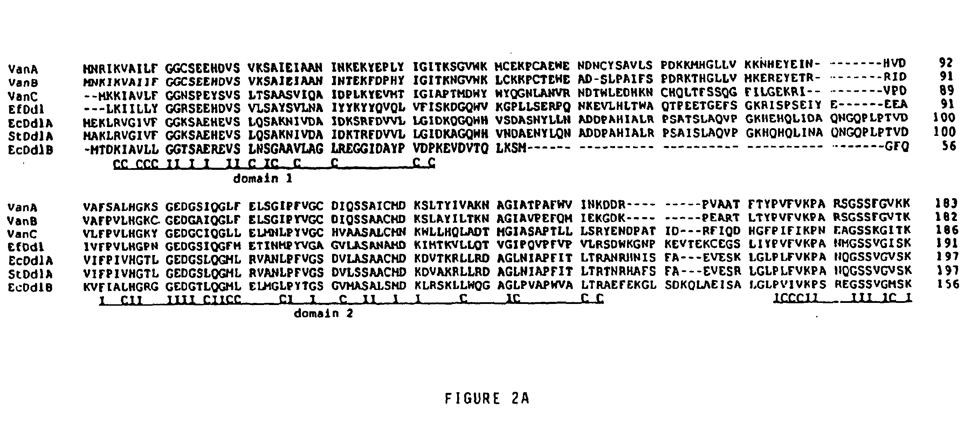

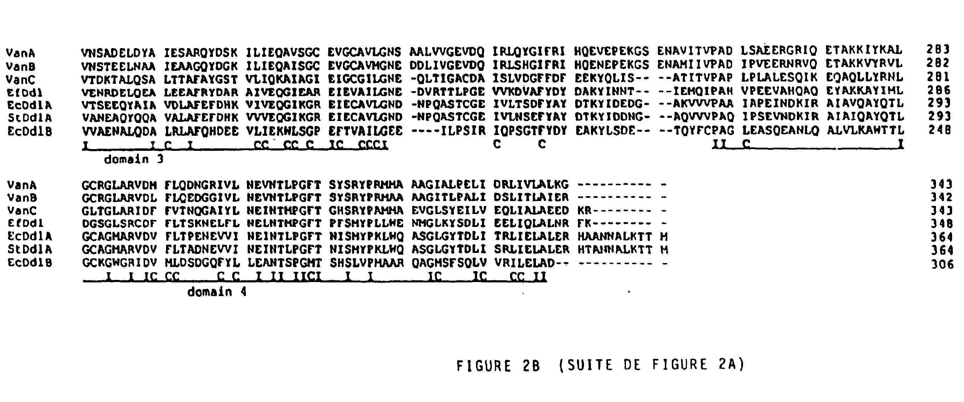

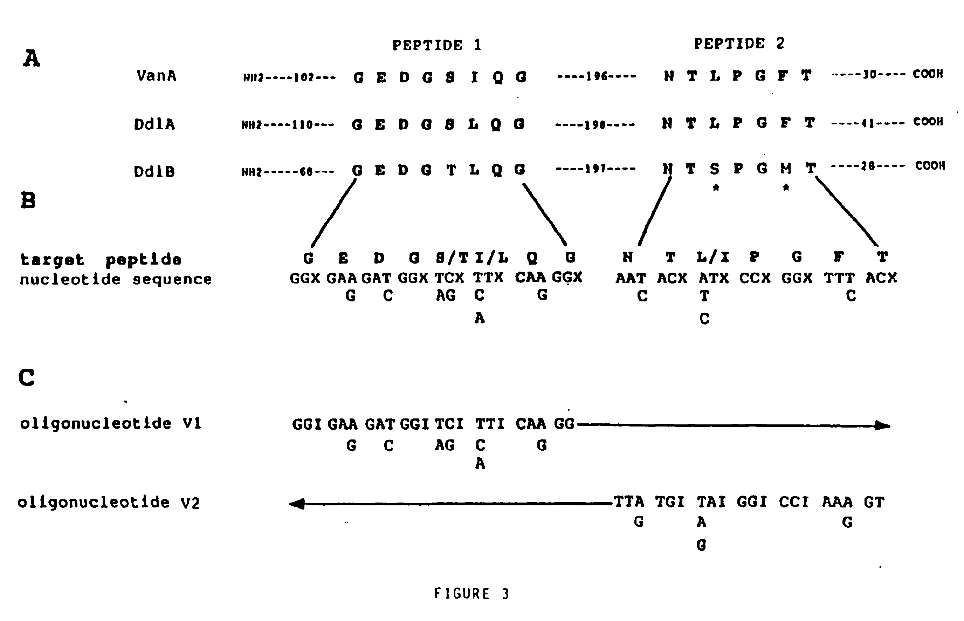

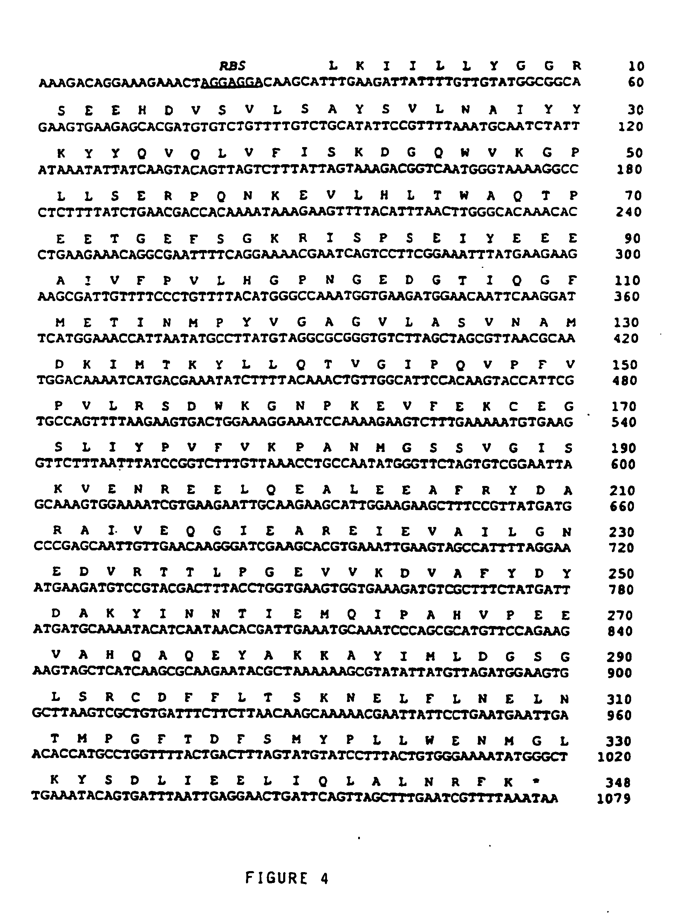

Selon un autre mode de réalisation, on détectera indifféremment une résistance correspondant à un phénotype VanA, VanB ou VanC, en utilisant une sonde commune aux gènes vanA, vanB ou vanC. Une telle sonde peut être réalisée à partir des séquences polypeptidiques alignées de la figure 2.

D'autres caractéristiques et avantages de l'invention apparaissent dans les exemples et les figures suivants :

### FIGURES

Figure 1 :
   Séquence de nucléotides et d'acides aminés correspondant au gène vanB. La séquence de nucléotides des deux brins a été déterminée à partir de l'insérat contenu dans pUC18, par la méthode didéoxy de terminaison de chaîne (Sanger et al, 1977, Proc. Natl. Acad. Sci. USA,74:5463-5467) en utilisant l'ADN polymérase T7.
   La séquence soulignée RBS représente la séquence Shine-Dalgarno de liaison au ribosome.
Figure 2 :
   Alignement de la séquence d'acides aminés déduite de la protéine VanB et des régions correspondantes de VanA, VanC, DdLA et DdLB de E.coli (Dutka-Malen et al, 1992 Gene, 112:53-58), Ddl de En.faecalis V583 et DdlA de S. typhimurium (Daub et al - Biochemistry 27, 1988, 3701-3708). Les acides aminés identiques (I) et les substitutions conservatives (C) dans les 7 séquences ont été indiqués sous l'alignement. Pour que la classification en tant que substitution soit conservée, les acides aminés ont été regroupés comme suit : RK, LFPMVI, STQNC, AGW, H, ED et Y.
   Les domaines 1, 2, 3 et 4 correspondent à des régions de forte homologie.
Figure 3 :
   Oligonucléotides V1 et V2 utilisés pour amplifier l'ADN du gène vanB.
Figure 4 :
   Séquence de nucléotides du gène ddl de En.faecalis V583 et séquence d'acides aminés correspondante. Le plasmide pAT203 a été construit en sous-clonant l'ADN d'un bactériophage - λ recombinant partiellement digéré avec Sau3AI (Pharmacia), dans pUC19 digéré avec BamHI (Pharmacia). L'insérat de 15 kb de pAT203 comprend le gène ddl. La séquence de nucléotides de 1079 pb consécutives de pAT203 a été déterminée sur les deux brins par la méthode dideoxy de terminaison de chaîne. La première paire de bases de la séquence est définie à la position 1. La séquence de liaison au ribosome RBS est à la position 19 en amont du codon de départ TTG. Le codon stop TTA est indiqué. La séquence d'acides aminés déduite de la protéine Ddl est présentée.

### APPROCHE EXPERIMENTALE

Les antibiotiques de la famille des glycopeptides, tels que la vancomycine (Vm) et la teicoplanine (Te) se fixent aux résidus D-Ala C-terminaux des précurseurs du peptidoglycane, bloquant ainsi leur incorporation dans la paroi cellulaire bactérienne (Reynolds, P.E. 1989 Eur. J. Clin. Microbiol. Infect. Dis. 8:943-950). Les résidus D-Ala sont incorporés dans les précurseurs au niveau de la paroi cellulaire, sous forme de dipeptides synthétisés par des ligases D-Ala:D-Ala (DDL) (Walsh, C.T. 1989 J. Biol. Chem. 264:2393-2396). La ligase VanA synthétise le dipeptide D-Ala-D-lac qui se substitue à D-Ala-D-Ala conduisant à la synthèse de précurseurs qui lient la vancomycine avec une affinité réduite (Bugg et al, Biochemistry 30:10408-10415 (1991), Handwerger et al, J. Bacteriol. 174:5982-5984 (1992), Messer et Reynolds, FEMS Microbiol. Letters 94:195-200 (1992)).

La résistance aux glycopeptides chez les entérocoques est hétérogène (Dutka-Malen et al, 1990 Antimicrobiol Agents Chemother 34:1875-1879).

Les protéines de résistance VanA et VanC (voir demande de brevet EP 91920753.0 du 29 octobre 1991) montrent une homologie de 30 à 37% (les détails sont donnés dans le tableau III) en acides aminés avec les D-Ala:D-Ala ligases (Ala=Alanine) de E.coli (Dutka-Malen et al, 1992 Gene 112:53-58). Les gènes de structure pour les protéines VanA et VanC n'hybrident pas avec l'ADN des souches de phénotype VanB (Dutka-Malen et al, 1990, Leclercq et al, Antimicrob. Agents Chemother 36:2005-2008 (1992)).

Les inventeurs sont parvenus à identifier la séquence nucléotidique impliquée dans les propriétés de résistance à la vancomycine de souches d'entérocoques ayant le phénotype VanB et résistant, après induction avec la vancomycine.

Souches Bactériennes : 39 isolats de E.faecium (28 souches) et E.faecalis (11 souches) résistantes à de faibles ou de fortes concentrations de vancomycine et sensibles à la teicoplanine ont été étudiées (tableau II). Parmi ces souches, 24 isolats y compris E.faecalis V583 (Sahm D. et al, Antimicrob. Agents Chemother. 1989, 33:1588-91) et E.faecium D366 (Gutmann L. et al, Antimicrob. Agents Chemother 1992, 36:77-80) étaient résistantes à de faibles concentrations de vancomycine sur la base d'un test de sensibilité sur disque. Ces souches appartiennent au phénotype de classe B. 15 isolats résistant à de fortes concentrations de vancomycine (CIM ≥ 128 µg/ml) y compris E.faecalis souche V583-2 (Zarlenga LJ. et al, Antimicrob. Agents Chemother. 1992, 36:902-5), qui est un mutant spontané de V553, ainsi que UMH-1 (Schwalbe R. et al, Abstract A-117, in Abstracts of the 91st General Meeting of the American Society for Microbiology. Dallas, TX: American Society for Microbiology, 1991) ont aussi été étudiés. Les souches-contrôle étaient des souches d'enterocoque bien caractérisées appartenant aux phénotypes A et C et hybridant avec les sondes VanA et VanC respectivement. Il s'agit en particulier de 6 isolats cliniques de E.faecium hautement résistants à la vancomycine et à la teicoplanine, incluant BM4147. Les souches de E. gallinarum incluant BM4174 appartenant à la classe C ont également été utilisées comme contrôle. Font également partie les contrôles de souches de E.casseliflavus, y compris la souche ATCC 25788, qui sont des isolats intrinsèquement résistants à de faibles niveaux de vancomycine et sensibles à la teicoplanine (Leclercq R. et al, Antimicrob. Agents Chemother. 1992, 36:2005-8).

Les souches suivantes ont également été étudiées : Erysipelothrix rhusiopathiae A 124 (Institut Pasteur Collection), Lactobacillus brevis ATCC 14869, Lactobacillus casei ATCC 393, Lactobacillus confusus ATCC 10881, Lactobacillus fermentum ATCC 9338, Lactobacillus plantarum ATCC 8014, Lactobacillus reuteri ATCC 23272, Lactobacillus rhamnosus ATCC 7469, Lactobacillus salivarius ATCC 11741, Pediococcus acidilacti ATCC 8042, Pediococcus pentosaceus ATCC 33316, et Leuconostoc mesenteroides CIP 16407. Les enterocoques suivants sensibles à des antibiotiques de la famille des glycopeptides sont utilisés comme contrôles négatifs: E.durans ATCC 19432, E.faecium ATCC 19434, BM4107 (Leclercq R. et al, Antimicrob. Agents Chemother. 1992, 36:2005-8), et MT10R (GutmannL et al, Antimicrob. Agents Chemother, 1992, 36:77-80), souche sensible à la vancomycine dérivée de D366 ; E.faecalis ATCC 29212, ATCC 33186,JH2-2 (Leclercq R. et al, Antimicrob. Agents Chemother. 1992, 36:2005-8), et V583-C1, souche sensible à la vancomycine dérivée de V583, (Tableau II), et un isolat clinique de E.faecium et E.faecalis. Des caractéristiques de souches de référence sont dépeintes au tableau I. E.faecium BM4107 et E.faecalis JH2-2, à la fois résistantes à la rifampine et à l'acide fusidique (Leclercq R. et al, Antimicrob. Agents Chemother. 1992, 36:2005-8), étaient utilisés comme souches réceptrices pour des expériences de conjugaison.

### Identification des entérocoques

Les entérocoques ont été identifiés par la méthode de Facklam et Collins, J. Clin. Microbiol. 1989, 27:731-4). L'identification des espèces était fondée sur les tests de réduction du tellurite de potassium et de production d'acides à partir de carbohydrates sur des bandes de streptocoques API 20 (bioMérieux, Marcy l'Etoile, France). Le test de mobilité à 30°C et de fermentation des carbohydrates ont été utilisés pour distinguer E. gallinarum et E.casseliflavus de E.faecium et E.faecalis. Les souches de E.casseliflavus ont été distinguées des souches de E.gallinarum sur la base de la production d'un pigment jaune sur de l'agar.

### Milieu

Un milieu coeur-cerveau et de l'agar (Difco Laboratoires, Détroit, MI) ont été utilisés. Des tests de sensibilité ont été réalisés sur de l'agar Mueller-Hinton (Diagnostics Pasteur, Marne La Coquette, France). Toutes les incubations ont été réalisées à 37°C.

### Détermination de la sensibilité in vitro aux antibiotiques.

Le test de diffusion sur disque avec des disques contenant 30 µg de vancomycine ou 30 µg de teicoplanine (Diagnostics Pasteur) a été utilisé pour le criblage initial. La méthode de Steers et al avec 10⁴ CFU par tâche a été utilisée pour déterminer la CMI des antibiotiques (Steers E. et al, Antibiot. Chemother. (Basel) 1959, 9:307-11).

### Transfert du caractère de résistance d'un antibiotique

La conjugaison sur filtres a été réalisée selon la technique décrite par Dutka-Malen S. et al, Antimicrob. Agents Chemother. 1990, 34:1875-9. Les concentrations en antibiotiques pour la sélection des transconjugués étaient les suivantes : rifampine : 20 µg/ml ; acide fusidique : 10µg/ml et vancomycine : 4 et 8 µg/ml.

### Enzymes et réactifs

La lisozyme a été obtenue chez Sigma Chemical Co. (St Louis, MO). La RNase A (pancréas de bovin) et la protéinase K ont été obtenues chez Calbiochem. Co. (San Diego, CA). L'[α-³²P]dCTP et le sel de triethylammonium (activité spécifique 3000 CI/mmol) ont été obtenus de Radiochemical Center, Amersham, Grande-Bretagne. La teicoplanine provient de Gruppo Lepetit (Milan, Italie) et la vancomycine provient de Eli Lilly & Co (Indianapolis, IN).

Les oligonucléotides V1 et V2 décrits dans la demande de brevet EP 91920753.0, ont permis l'amplification par la technique de PCR, de fragments internes aux gènes codant pour les protéines VanA, VanC et D-Ala:D-Ala ligases (Dutka-Malen et al, 1992 Gene 112:53-58).

L'amplification du gène vanB a été réalisée avec les oligonucléotides V1 et V2 et l'ADN (20 ng) de Enterococcus faecalis V583 (Sahm et al, 1989 Antimicrob. Agents Chemother 33:1588-1591).

Pour réaliser cette amplification, la technique décrite dans la publication de Dutka-Malen et al., 1992 a été utilisée. Les fragments obtenus ont été séparés sur gel d'agarose (1%) dans un tampon TAE, qui a permis de révéler une bande unique d'environ 600 pb, qui a été extraite du gel en utilisant un kit de purification de l'ADN (GeneClean, Bio101 Inc., la Jolla, CA). En utilisant un kit permettant l'obtention d'extrémités franches au niveau de l'ADN (Amersham, Amersham, Grande-Bretagne), les fragments ont été traités avec l'ADN T4 polymérase et ligaturés au niveau du site SmaI d'un plasmide pUC18 digéré et déphosphorilé (Norrander et al, 1983, Gene 26:101-106).

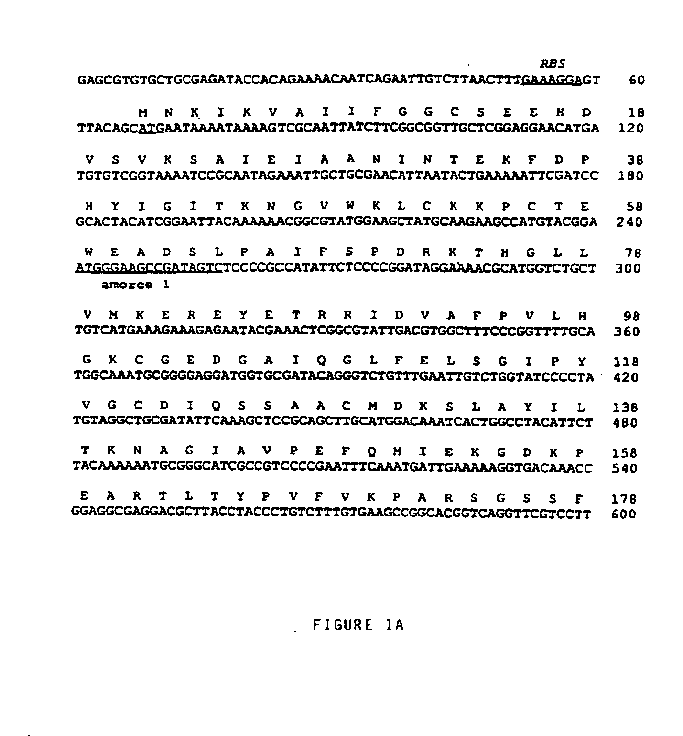

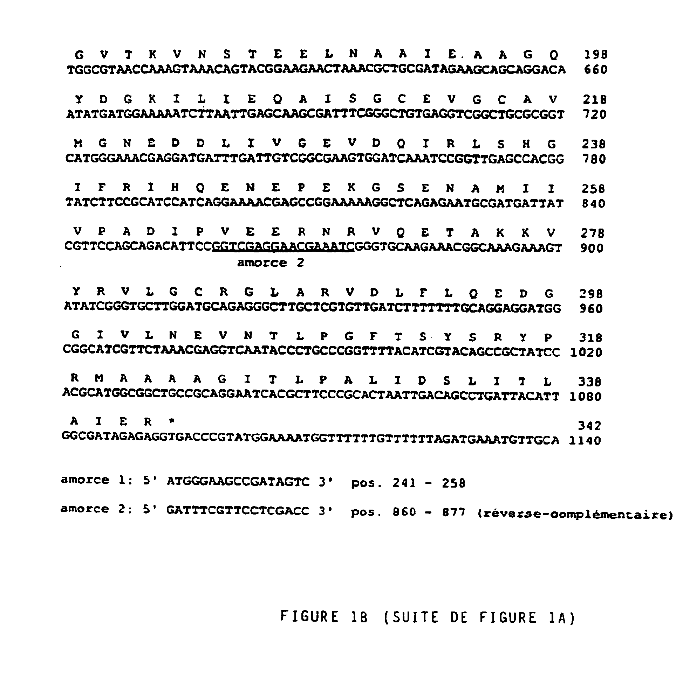

La séquence de 632 pb (sonde vanB) correspondant à l'insert du plasmide recombinant (figure 1) a été déterminée par la méthode didéoxy de terminaison de chaîne (Sanger et al, 1977, Proc. Natl. Acad. Sci. USA, 74:5463-5467) en utilisant l'ADN T7 polymérase (Pharmacia, Uppsala, Suède) et le [α-³⁵S]dATP (Amersham, Radiochemical center, Amersham, Grande-Bretagne).

Etant donné que l'amplification avec l'ADN Taq polymérase peut conduire à des incorporations erronées de nucléotides, la séquence a été confirmée comme suit : un oligonucléotide complémentaire des positions 513 à 530 de la séquence nucléotidique de la figure 1 a été synthétisé par la méthode au phosphoramidite (unité de Chimie organique, Institut Pasteur, France) et utilisé avec l'amorce V1 pour réaliser une amplification par PCR d'un fragment de vanB. Le produit PCR a été séquence directement (Mabilat et al, 1990, Plasmid 23:27-34) ou après le clonage dans un vecteur pUC18, afin de révéler l'identité des nucléotides avec le fragment clone obtenu avec V1 et V2.

Une hybridation de type southern a été réalisée selon la méthode de Johnson et al, Gene Anal. Téchnol. 1:3-8 (1984). l'ADN total des souches d'entérocoque (Tableau 1) a été préparé selon la technique décrite par Le Bouguénec et al, 1990, J. Bacteriol. 172:727-734, digéré avec les enzymes HindIII et KpnI (United States, Biochemical Corporation, Cleveland, Ohio) et résolu sur des gels d'agarose 1% . L'ADN a été transféré sur des membranes de nylon (Nytran, Schleicher & Schuell, Dassel, Allemagne) avec un appareil de transfert sous vide (Trans. Vac. TE80, Hoefer Scientific Instruments, San Francisco, CA). La sonde a été obtenue en marquant le fragment PCR cloné avec un kit de translation de césure (Bethesda Research Laboratories Life Technologies Inc., Gaithersburg, MD) et (α-³²P)dCTP (Amersham, Radiochemical Center, Amersham, Grande-Bretagne). L'hybridation a été réalisée dans des conditions stringentes à 68°C (Johnson et al, 1984, Gene Anal. Technol. 1:3-8). Les membranes ont été lavées à 65°C dans 0,1% SDS-2XSSC.

La sonde vanA consistait en un fragment PstI de 265 pb interne au gène vanA (Dukta Malen S. et al, Mol. Gen. Genet. 1990, 224:364-72). La sonde vanC consistait en un fragment EcoRI-HincII de 690 pb, interne au gène vanC (Leclercq R. et al, Antimicroh. Agents Chemother 1992, 36:2005-8. Dukta Malen S. et al Gene, 1992, 112:53-8). La sonde vanB correspond à la séquence II.

La séquence en acides aminés déduite de l'insérat contenu dans le plasmide pUC18 a été comparée avec différentes séquences de protéines [figure 2] : le tableau 5 résume les pourcentages d'identité en acides aminés comparés deux à deux pour les séquences des protéines VanB, VanA, VanC, ElDdl, DdlA et DdlB.
Dans les conditions d'hybridation en southern, le fragment clone hybridait avec le fragment de 3,3 kb HindIII-KpnI de E. faecalis V583. La sonde n'hybride pas avec l'ADN d'un dérivé de V583 sensible à la vancomycine ou à l'ADN des souches de E. faecalis et E. faecium sensibles à la vancomycine utilisées comme référence. Le fragment d'ADN clone obtenu par PCR correspond à un fragment interne du gène impliqué dans la résistance. Ce gène code pour une enzyme apparentée aux D-Ala:D-Ala ligases, appelée VanB, qui pourrait être impliquée dans la synthèse d'un produit se substituant à D-Ala-D-Ala.

Ces tests ont permis de mettre en évidence un seul groupe de gènes apparentés à vanB, et responsable d'un bas et d'un haut niveau de résistance à la vancomycine chez les entérocoques (tableaux 1 et 2).

Aucune hybridation n'a été observée entre la sonde VanB et l'ADN de souches sensibles à la vancomycine sans induction ou portant les gènes vanA ou vanC ou intrinsèquement résistantes.

La séquence complète du gène vanB a été clonée en mettant en oeuvre les étapes suivantes :

Le plasmide pAT202 a été obtenu en sous-clonant dans pUC19 un fragment HindIII-KpnI de 3,3 kb du bactériophage recombinant λ contenant le gène vanB. Le clonage a été réalisé avec des endonucléases de restriction (Boehringer, Mannheim, Allemagne et Pharmacia LKB Biotechnology Inc. Uppsala, Suède), la T4 DNA ligase (Boehringer) et la phosphatase alkaline (Pharmacia) conformément aux recommendations du fabricant. La séquence nucléotidique de 1090 pb consécutives de pAT202 a été déterminée pour les deux brins par la méthode dideoxy de terminaison de chaine (Sanger et al, 1977) en utilisant la T7 DNA polymérase modifiée (Amersham Radiochemical Center, Amersham, Grande-Bretagne) et des oligonucléotides complémentaires de la séquence, synthétisés par la méthode au méthoxy phosphoramidite (Institut Pasteur, Paris, France). Les produits des réactions ont été résolus par électrophorèse sur gel de polyacrylamide 6% dénaturant. La première paire de bases de la séquence présentée correspond à la position 1 (figure 1). Le site potentiel de liaison au ribosome (RBS) (Moran et al, Mol. Gen. Genet. 186 (1982) 339-346) en amont du codon ATG d'initiation à la position 46 est souligné. Le codon stop (TGA) est indiqué par une astérisque. La séquence d'acides aminés est alignée avec le premier nucléotide de chaque codon.

Le transfert du caractère de résistance à la vancomycine (chez 6 isolats d'entérocoques parmi 17) par conjugaison sur filtre, a été observé chez des souches E.faecium et E.faecalis résistantes à de faibles ou à de fortes concentrations d'antibiotiques.

Parmi les autres fragments d'environ 600 bp amplifiés à partir des oligonucléotides V1 et V2, un insérat hybridait avec l'ADN de souches Vm^{R} ou Vm^{S} de En.faecalis mais pas avec l'ADN de souches de 18 autres espèces. Ce gène code pour une D-Ala:D-Ala ligase dans En.faecalis. Puisqu'aucun autre gène de ligase dans En.faecalis n'a été détecté, ce gène a été appelé ddl.

Le clonaqe et le séquençage du gène ddl inséré dans le vecteur pUC19 (Norrander et al, Gene 26, 1983, 101-106) ont permis de constater que la teneur en bases G et C de ddl (37,5%) et du chromosome de En.faecalis (37-39%) étaient très semblables.

Différentes observations suggèrent que le gène vanB aurait une origine exogène : (i) le gène peut être transféré par conjugaison. (ii) Les séquences de nucléotides apparentées à van B n'ont pas été détectées dans l'ADN de souches Vm^{S} de En.faecalis et En.faecium et les représentants de 16 autres espèces d'Enterococcus (tableau III). (iii) La teneur en bases GC du gène vanB diffère de façon marquée de celle du chromosome de En.faecalis. (iv) Le niveau faible de similitude entre Ddl de En.faecalis et VanB (34% d'identité) indique que les gènes correspondants n'ont pas pour origine une duplication récente.

### Precurseurs de peptidoglycanes chez En. faecalis Vm^{R} et Vm^{S}

L'incubation de En.faecalis V583 avant l'induction de souches En.faecalis JH2-2 Vm^{R} ou Vm^{S} (Jacob et Hobbs - J. Bacteriol. 117, 1974, 360-372) avec la ramoplanine (9 µg/ml) inhibiteur de la paroi cellulaire a conduit à l'accumulation du précurseur de la paroi cellulaire UDP-N-acetylmuramyl-L-Ala-D-Glu-L-Lys-D-Ala-D-Ala (UDP-Mur-NAc-pentapeptide) qui est utilisé dans le cycle normal de la synthèse du peptidoglycane.

Après l'induction de la résistance, En.faecalis V583 a accumulé trois intermédiaires de la paroi cellulaire lorsque la souche était incubée avec la ramoplanine (tableau IV). Ces intermédiaires ont été identifiés comme étant des UDP-MurNAc-pentapeptides, UDP-MurNAc-tetrapeptide dépourvu de la partie c-terminale D-Ala de l'UDP-MurNAc-pentapeptide ; et de façon prédominante, UDP-MurNAc-tetrapeptide-D-lactate dans lequel la partie C-terminale D-Ala de l'UDP-MurNAc-pentapeptide est remplacée par le D-lactate. La présence de UDP-MurNAc-tetrapeptide-D-lactate suggère que les souches de phénotypes VanA et VanB ont le même mécanisme de base de résistance aux glycopeptides ; c'est à dire qu'ils synthétisent du D-lactate qui peut être lié à VanB (ou VanA) par le D-Ala pour synthétiser du D-Ala-D-lactate qui est ensuite incorporé dans le précurseur de peptidoglycane.

Les précurseurs de la paroi ont été purifiés par chromatographie d'échange d'ions et désalage par filtration sur gel. L'identification a été fondée sur une spectroscopie de masse (positive ion electrospray mass spectroscopy), un spectre UV (pour l'uracil) analyse automatisée des acides aminés après hydrolyse pendant 4h et 24h (pour l'acide muramique et les taux en acides aminés) et l'analyse par réactions enzymatiques spécifiques du résidu terminal obtenu par réaction avec le D,D-carboxypeptidase de Actinomadura R39 (Messer et Reynolds, FEMS Microbiol Letter 94, 1992, 195-200).

La quantité totale de précurseurs accumulée dans chaque culture était semblable approximativement. 65 µmol/g de poids sec dans une période d'incubation correspondant à 0,6 de la durée moyenne de synthèse.

**TABLEAU I: Souches bactériennes**

| | CMI (µg/ml) | | Hybridation avec la sonde vanB | | | |
|---|---|---|---|---|---|---|
| Souche | Vm | Te | | | | Référence |
| | | | vanA | vanB | vanC | |
| E. faecalis | | | | | | |
| V583 | 64 | 0,5 | - | + | - | Sahm et al (1989) |
| V583-C1 | 2 | 0,5 | - | - | - | D.F. Sahm |
| V583-2 | 1024 | 1,0 | - | + | - | Zarlenga LJ (1992) |
| UMH-1 | 1024 | 1,0 | - | + | - | Schwalbe et al (1991) |
| ATCC 29212 | 2 | 0,5 | - | - | - | |
| | | | | | | |
| E.faecium | | | | | | |
| D366 | 32 | 0,5 | - | + | - | Gutmann et al (1992) |
| MT10R | 2 | 0,25 | - | - | - | Gutmann et al (1992) |
| BM4147 | 1024 | 512 | + | - | - | Dutka-Malen et al (1990) |
| ATCC 19434 | 1 | 1 | - | - | - | |
| | | | | | | |
| E. gallinarum BM4174 | 8 | 1 | - | - | + | Dutka-Malen et al (1992) |
| | | | | | | |
| E.casseliflavus | 8 | 1 | - | - | - | |

**TABLEAU II:**

| **Classes de phénotypes et génotypes parmi les coccins à Gram-positif, résistantes à la vancomycine** | | | | |
|---|---|---|---|---|
| CLASSE DE PHENOTYPE | CLASSE DE (A) GENOTYPE | ESPECE | CMI (µg/ml) | |
| | | | Vancomycine | Teicoplanine |
| Susceptible | Susceptible | Enterococcus spp.(10) | 0.5-2 | 0.25 |
| A | A | E.faecium(6) | 256->1.000 | 64->1.000 |
| B | B | E.faecium(28) | 4-256 | 0.5-1 |
| B | B | E.faecalis(11) | 4-1024 | 0.5-1 |
| C | C | E.gallinarum(3) | 8 | 1 |
| C | NC | E.casseliflavus(2) | 4-8 | 0.5-1 |
| NC | NC | Lactobacillus spp.(8) | >1.000 | >1.000 |
| NC | NC | Leuconostoc. sp.(1) | >1.000 | >1.000 |
| NC | NC | Pediococcus spp.(2) | >1.000 | >1.000 |
| NC | NC | E.rhusiopathiae(1) | >1.000 | >1.000 |

| | | | | |
|---|---|---|---|---|
| ^{(a)}A: hybridation avec la sonde vanA ; B: hybridation avec la sonde vanB ; C: hybridation avec la sonde vanC ; NC : non classé | | | | |

**TABLEAU III**

| Résultats des expériences d'hybridation | | | | |
|---|---|---|---|---|
| | Resistance phenotype | Nbre de souches testées | Hybridation avec sonde | |
| Espèces | | | *vanB* | *ddl (En. faecalis)* |
| *En. faecalis* | Vm^{R}, Te^{S} | 11 | + | + |
| | Vm^{S}, Te^{S} | 5 | - | + |
| *En. faecium* | Vm^{R}, Te^{R} | 6 | - | - |
| | Vm^{R}, Te^{S} | 28 | + | - |
| | Vm^{S}, Te^{S} | 4 | - | - |
| *En. gallinarum* | Vm^{R}, Te^{S} | 3 | . | - |
| *En. carselifiavus* | Vm^{R}, Te^{S} | 2 | - | - |
| *En.* spp. (15 espèces^{a}) | Vm^{S}, Te^{S} | 15 | - | - |

| | | | | |
|---|---|---|---|---|
| ^{a} Type de souches de En.avium, En. cecorum, En. *columbae. En. dispar, En. durans, En. flavescens. En. hirae, En. malodoratus, En. mundtii, En. pseudoavium. En. raffinosus, En. saccharolyticus. En*. *seriolicida, En. solitarius et En. sulfureus.* | | | | |

**TABLEAU IV**

| Précurseurs de peptidoglycane dans des souches de En. Faecalis Vm^{S} ou Vm^{R} | | | |
|---|---|---|---|
| Précurseur de Peptidoglycane | Quantité de précurseur (%) de En. faecalis | | |
| | JH2-2 | V583 non-induite | V583 induite |
| UDP-MurNAc-L-Ala-D-Glu-L-Lys-D-Ala-D-Ala | 100 | 100 | 14 |
| UDP-MurNAc-L-Ala-D-Glu-L-Lys-D-Ala | 0 | 0 | 7 |
| UDP-MurNAc-L-Ala-D-Glu-L-Lys-D-Ala-D-lactate | 0 | 0 | 79 |

**TABLEAU V**

| Identité de séquence entre les séquences d'acides aminés de Van b, Ddl de En.faecalis V583 et des D-Ala:D-Ala ligases^{a} | | | | | | |
|---|---|---|---|---|---|---|
| Sequence comparée^{b} | Pourcentage d'identité par rapport à : | | | | | |
| | VanB | VanC | EfDdl | EcDdlA | StDdlA | EcDdlB |
| VanA | 76 | 38 | 32 | 38 | 37 | 30 |
| VanB | | 38 | 34 | 38 | 38 | 32 |
| VanC | | | 34 | 34 | 34 | 36 |
| EfDdl | | | | 40 | 40 | 34 |
| EcDdlA | | | | | 90 | 35 |
| StDdlA | | | | | | 35 |

| | | | | | | |
|---|---|---|---|---|---|---|
| ^{a} Identité des paires de séquences dérivée de l'alignement de la figure 2 ^{b} Ec, E.coli; Ef, En.faecalis; St, S.typhimurium | | | | | | |

## Revendications

1. Protéine VanB **caractérisée en ce qu'**elle comprend la séquence d'acides aminés suivante, et **en ce qu'**elle est impliquée chez des bactéries à Gram-positif, dans une résistance à la vancomycine, cette résistance étant de type inductible par la vancomycine et non inductible par la teicoplanine.

2. Protéine VanB selon la revendication 1, **caractérisée en ce qu'**elle a la capacité de conférer une résistance de type inductible à la vancomycine, chez des entérocoques et par exemple chez des souches du genre *E. faecium* ou *E. faecalis*.

3. Protéine VanB **caractérisée en ce qu'**elle comprend une séquence d'acides aminés modifiée par rapport à la séquence telle que définie à la revendication 1 par délétion ou insertion ou remplacement d'un ou plusieurs acides aminés, dès lors que la protéine VanB ainsi modifiée est nécessaire à l'établissement d'une résistance à la vancomycine chez des bactéries gram-positif, cette résistance étant de type inductible par la vancomycine et non inductible par la teicoplanine.

4. Fragment peptidique de la protéine VanB, **caractérisé en ce qu'**il correspond à la séquence d'acides aminés telle que définie à la revendication 1 ou à toute partie de cette séquence suffisante pour conférer la résistance à la vancomycine, chez des bactéries à Gram-positif, par exemple des bactéries de la famille des entérocoques, cette résistance étant de type inductible par la vancomycine et non inductible par la teicoplanine.

5. Fragment peptidique de la protéine VanB, **caractérisé en ce qu'**il correspond à la séquence d'acides aminés telle que définie à la revendication 1 ou à toute partie de cette séquence et **en ce qu'**il est reconnu spécifiquement par des anticorps dirigés contre la protéine VanB, et qu'il est dépourvu de réaction immunologique avec des anticorps reconnaissant les protéines VanA et/ou VanC.

6. Séquence de nucléotides **caractérisée en ce qu'**elle code pour une protéine VanB impliquée chez des bactéries à Gram-positif, dans une résistance à la vancomycine, cette résistance étant de type inductible par la vancomycine et non inductible par la teicoplanine, ladite protéine VanB comprenant dans son ossature peptidique la séquence d'acides aminés 1 définie à la revendication 1, ou **en ce qu'**il s'agit d'une séquence d'ADN complémentaire de cette séquence codante, ou d'une séquence d'ARN correspondante.

7. Séquence de nucléotides selon la revendication 6, **caractérisée en ce qu'**elle comprend l'enchaînement de nucléotides ci-dessous ou un enchaînement nucléotidique modifié par mutation, addition ou délétion de nucléotides par rapport à l'enchaînement de nucléotides ci-dessous, dès lors qu'il code pour une protéine impliquée chez des bactéries à Gram-positif, dans une résistance à la vancomycine, cette résistance étant de type inductible par la vancomycine et non inductible par la teicoplanine.

8. Fragment nucléotidique, **caractérisé en ce qu'**il est capable d'hybrider dans des conditions stringentes avec une séquence selon l'une quelconque des revendications 6 ou 7 ou **en ce qu'**il est complémentaire au fragment nucléotidique hybridant, ledit fragment :
(a) codant un fragment de VanB, définie selon l'une quelconque des revendications 1 à 3, suffisant pour conférer la résistance à la vancomycine, cette résistance étant de type inductible par la vancomycine et non inductible par la teicoplanine ou,
(b) permettant la détection spécifique de séquences codant le gène vanB, défini dans la revendication 6 ou 7.

9. Fragment nucléotidique selon la revendication 8, **caractérisé en ce qu'**il n'hybride pas dans des conditions stringentes avec l'ADN de souches d'entérocoques sensibles à la vancomycine, en particulier avec l'ADN des souches *E. faecalis* JH2-2, *E. faecium* BM4107.

10. Fragment nucléotidique selon l'une quelconque des revendications 8 ou 9, **caractérisé en ce que** sa séquence consiste en l'enchaînement nucléotidique illustré dans la revendication 7.

11. Fragment nucléotidique selon l'une quelconque des revendications 8 à 10, **caractérisé en ce qu'**il est marqué.

12. Fragment nucléotidique selon la revendication 8, **caractérisé en ce qu'**il s'agit de l'une des séquences suivantes :
- amorce 1 : 5' ATGGGAAGCCGATAGTC 3'
- amorce 2 : 5' GATTTCGTTCCTCGACC 3'.

13. Séquence d'ADN recombinante, **caractérisée en ce qu'**elle comprend une séquence de nucléotides selon l'une quelconque des revendications 6 à 11, sous le contrôle d'éléments de régulation susceptibles d'intervenir dans l'expression d'une résistance à la vancomycine, chez un hôte déterminé, cette résistance étant inductible par la vancomycine et non inductible par la teicoplanine.

14. Vecteur recombinant pour le clonage ou l'expression dans un hôte déterminé, **caractérisé en ce qu'**il comprend une séquence nucléotidique selon l'une quelconque des revendications 6 à 11, en un site non essentiel pour sa réplication, sous le contrôle d'éléments de régulation susceptibles d'intervenir dans l'expression d'une résistance à la vancomycine, cette résistance étant inductible par la vancomycine et non inductible par la teicoplanine.

15. Vecteur recombinant selon la revendication 14, **caractérisé en ce qu'**il s'agit du plasmide pAT201 déposé à la CNCM sous le numéro I-1277 ou du plasmide pAT202 déposé à la C.N.C.M. sous le numéro I-9291.

16. Hôte cellulaire recombinant, **caractérisé en ce qu'**il comprend une séquence nucléotidique selon l'une quelconque des revendications 6 à 11, ou un vecteur selon la revendication 14 ou la revendication 15, cet hôte étant choisi parmi les bactéries, notamment les cocci à Gram-positif.

17. Anticorps monoclonaux ou polyclonaux **caractérisés en ce qu'**ils reconnaissent spécifiquement la protéine VanB selon l'une quelconque des revendications 1 à 3 ou un fragment peptidique selon l'une quelconque des revendications 4 à 5.

18. Kit pour le diagnostic *in vitro* sur un échantillon biologique, de la présence de souches résistantes à la vancomycine, ces souches appartenant aux cocci à Gram-positif, notamment en ce qu'il s'agit des souches d'entérocoques, par exemple *E*. *faecium,* **caractérisé en ce qu'**il comprend :
- des anticorps selon la revendication 17, le cas échéant marqués,
- un réactif pour la détection d'une réaction immunologique du type anticorps-antigène,
- le cas échéant des réactifs pour effectuer la lyse des cellules de l'échantillon testé,
- le cas échéant une concentration déterminée de vancomycine pour induire la résistance.

19. Kit selon la revendication 18, **caractérisé en ce qu'**il comprend en outre des anticorps dirigés spécifiquement contre la protéine VanA et/ou des anticorps dirigés spécifiquement contre la protéine VanC.

20. Kit pour le diagnostic *in vitro* de la présence de souches résistantes à la vancomycine, ces souches appartenant en particulier aux cocci à Gram-positif, notamment en ce qu'il s'agit de souches d'entérocoques, par exemple *E. faecium,* **caractérisé en ce qu'**il comprend :
- une sonde nucléotidique selon la revendication 11, et le cas échéant,
- des nucléoside-triphosphates dATP, dCTP, dTTP, dGTP,
- un agent de polymérisation d'ADN.

21. Kit selon la revendication 20, **caractérisé en qu'**il comprend en outre des sondes nucléotidiques spécifiques des gènes vanA et/ou vanC.

22. Procédé de détection *in vitro* de la présence de souches résistantes à la vancomycine et/ou à la teicoplanine, ces souches appartenant à la famille des cocci à Gram-positif, notamment en ce qu'il s'agit de souches d'entérocoques, par exemple *E. faecium* ou *E. faecelis,* **caractérisé en ce qu'**il comprend :
a) la mise en contact d'un échantillon biologique susceptible de contenir les souches résistantes, avec une amorce constituée par un fragment nucléotidique selon l'une quelconque des revendications 8 ou 9, capable d'hybrider avec une séquence nucléotidique recherchée, nécessaire à l'expression de la résistance, cette séquence étant utilisée comme matrice, en présence des 4 différents nucléosides triphosphates, et d'un agent de polymérisation, dans des conditions d'hybridation telles que pour chaque séquence nucléotidique ayant hybridé avec une amorce, un produit d'élongation de chaque amorce complémentaire de la matrice est synthétisé,
b) la séparation de la matrice et du produit d'élongation obtenu, ce dernier pouvant alors également se comporter comme une matrice,
c) la répétition de l'étape a) de façon à obtenir une quantité détectable des séquences nucléotidiques recherchées,
d) la détection du produit d'amplification des séquences nucléotidiques.

23. Procédé de détection *in vitro* de souches résistantes à la vancomycine et/ou à la teicoplanine, ces souches appartenant à la famille des cocci à Gram-positif, notamment en ce qu'il s'agit de souches d'entérocoques, par exemple *E. faecium* ou *E. faecalis,* **caractérisé en ce qu'**il comprend :
a) la mise en contact d'un échantillon biologique susceptible de contenir les souches résistantes, avec une amorce constituée par un fragment nucléotidique selon l'une quelconque des revendications 8 ou 9, et avec un fragment nucléotidique du gène vanA et un fragment nucléotidique spécifique du gène vanC, capable d'hybrider avec une séquence nucléotidique recherchée, nécessaire à l'expression de la résistance, cette séquence étant utilisée comme matrice, en présence des 4 différents nucléosides triphosphates, et d'un agent de polymérisation, dans des conditions d'hybridation telles que pour chaque séquence nucléotidique ayant hybridé avec une amorce, un produit d'élongation de chaque amorce complémentaire de la matrice est synthétisé,
b) la séparation de la matrice et du produit d'élongation obtenu, ce dernier pouvant alors également se comporter comme une matrice,
c) la répétition de l'étape a) de façon à obtenir une quantité détectable des séquences nucléotidiques recherchées,
d) la détection du produit d'amplification des séquences nucléotidiques.

## Claims

1. A VanB protein, **characterized in that** it comprises the following amino acid sequence and **in that** it is involved in vancomycin resistance in gram positive bacteria, said resistance being of the type which is inducible by vancomycin and not inducible by teicoplanin:

2. A VanB protein according to claim 1, **characterized in that** it has the capacity to endow a resistance of the vancomycin-inducible type in enterococci, for example in strains of the genus *E. faecium* or *E.faecalis.*

3. A VanB protein, **characterized in that** it comprises an amino acid sequence modified with respect to the sequence as defined in claim 1, by deletion or insertion or replacement of one or more amino acids, provided that the modified VanB protein is necessary for establishing vancomycin resistance in gram positive bacteria, said resistance being of the type which is inducible by vancomycin and not inducible by teicoplanin.

4. A peptide fragment of the VanB protein, **characterized in that** it corresponds to the amino acid sequence as defined in claim 1 or to any part of said sequence which is sufficient to endow vancomycin resistance in gram positive bacteria, for example bacteria from the enterococcus family, said resistance being of the type which is inducible by vancomycin and not inducible by teicoplanin.

5. A peptide fragment of the VanB protein, **characterized in that** it corresponds to the amino acid sequence as defined in claim 1 or to any part of that sequence and **in that** it is specifically recognized by antibodies directed against the VanB protein, and **in that** it is free of immunological reaction with antibodies recognizing VanA and/or VanC proteins.

6. A nucleotide sequence, **characterized in that** it codes for a VanB protein involved in vancomycin resistance in gram positive bacteria, said resistance being of the type which is inducible by vancomycin and not inducible by teicoplanin, said VanB protein comprising in its peptide backbone the amino acid sequence as defined in claim 1, or **in that** it is a complementary DNA sequence of said coding sequence, or a corresponding RNA sequence.

7. A nucleotide sequence according to claim 6, **characterized in that** it comprises the following concatenation of nucleotides or a nucleotide concatenation modified by mutation, addition or deletion of nucleotides compared with the following concatenation of nucleotides, provided that it codes for a protein involved in vancomycin resistance in gram positive bacteria, said resistance being of the type which is inducible by vancomycin and not inducible by teicoplanin:

8. A nucleotide fragment, **characterized in that** it is capable of hybridizing under stringent conditions with a sequence according to claim 6 or 7 or **in that** it is complementary to the hybridizing nucleotide fragment, said fragment:
a) coding for a VanB fragment as defined in any one of claims 1 to 3, sufficient to endow vancomycin resistance, said resistance being of the type which is inducible by vancomycin and not inducible by teicoplanin; or
b) allowing specific detection of sequences coding for the VanB gene, defined in claim 6 or 7.

9. A nucleotide fragment according to claim 8, **characterized in that** it does not hybridize under stringent conditions with DNA from enterococcus strains sensitive to vancomycin, in particular with DNA from the *E. faecalis JH2-2, E. faecium* BM4107 strains.

10. A nucleotide fragment according to claim 8 or 9, **characterized in that** its sequence consists in the nucleotide concatenation illustrated in claim 7.

11. A nucleotide fragment according to any one of claims 8 to 10, **characterized in that** it is labeled.

12. A nucleotide fragment according to claim 8, **characterized in that** it is one of the following sequences:
• primer 1: 5' ATGGGAAGCCGATAGTC 3'
• primer 2: 5' GATTTCGTTCCTCGACC 3'

13. A recombinant DNA sequence, **characterized in that** it comprises a nucleotide sequence according to any one of claims 6 to 11, under the control of regulating elements which may be involved in the expression of vancomycin resistance, in a given host, said resistance being inducible by vancomycin and not inducible by teicoplanin.

14. A recombinant vector for cloning or expression in a given host, **characterized in that** it comprises a nucleotide sequence according to any one of claims 6 to 11, in a site which is not essential for its replication, under the control of regulation elements which may be involved in the expression of vancomycin resistance, said resistance being inducible by vancomycin and not inducible by teicoplanin.

15. A recombinant vector according to claim 14, **characterized in that** it is the pAT201 plasmid deposited at the CNCM under number I-1277 or the pAT202 plasmid at the CNCM under number I-1291.

16. A recombinant host cell, **characterized in that** it comprises a nucleotide sequence according to any one of claims 6 to 11, or a vector according to claim 14 or claim 15, said host being selected from bacteria, in particular gram positive cocci.

17. Monoclonal or polyclonal antibodies, **characterized in that** they specifically recognize the VanB protein of any one of claims 1 to 3 or a peptide fragment according to any one of claims 4 to 5.

18. A kit for *in vitro* diagnosis, on a biological sample, of the presence of vancomycin-resistant strains, said strains belonging to gram positive cocci, in particular enterococcus strains, for example *E. faecium,* **characterized in that** it comprises:
• antibodies according to claim 17, optionally labelled;
• a reagent for detecting an immunological antibody-antigen type reaction;
• if appropriate, reagents to lyse cells of the test sample;
• if appropriate, a predetermined concentration of vancomycin to induce resistance.

19. A kit according to claim 18, **characterized in that** it further comprises antibodies specifically directed against the VanA protein and/or antibodies specifically directed against the VanC protein.

20. A kit for *in vitro* diagnosis of the presence of vancomycin-resistant claims, said strains belonging in particular to gram positive cocci, especially enterococcus strains, for example *E. faecium,* **characterized in that** it comprises:
• a nucleotide probe according to claim 11; and if appropriate
• nucleoside triphosphates dATP, dCTP, dTTP, dGTP;
• a DNA polymerization agent.

21. A kit according to claim 20, **characterized in that** it further comprises nucleotide probes specific for the VanA and/or VanC genes.

22. A method for *in vitro* detection of the presence of strains resistant to vancomycin and/or teicoplanin, said strains belonging to the gram positive cocci family, especially enterococcus strains, for example *E. faecium* or *E. faecalis,* **characterized in that** it comprises:
(a) bringing a biological sample which may contain resistant strains into contact with a primer constituted by a nucleotide fragment according to claim 8 or claim 9, capable of hybridizing with a desired nucleotide sequence necessary for the expression of resistance, said sequence being used as a matrix, in the presence of the 4 different nucleotide triphosphates and a polymerization agent, under hybridization conditions such that for each nucleotide sequence which has hybridized with a primer, an elongation product of each complementary primer of the matrix is synthesized;
(b) separating the matrix and the elongation product obtained, said elongation product thus also possibly behaving as a matrix;
(c) repeating step a) to obtain a detectable quantity of the desired nucleotide sequences; and
(d) detecting the amplification product of the nucleotide sequences.

23. A method for *in vitro* detection of strains resistant to vancomycin and/or teicoplanin, said strains belonging to the gram positive cocci family, **characterized in that** they are enterococcus strains, for example *E. faecium* or *E. faecalis,* **characterized in that** it comprises:
(a) bringing a biological sample which may contain resistant strains into contact with a primer constituted by a nucleotide fragment according to claim 8 or claim 9, and with a nucleotide fragment of the VanA gene and a specific nucleotide fragment of the VanC gene, capable of hybridizing with a desired nucleotide sequence necessary for the expression of the resistance, said sequence being used as a matrix, in the presence of the 4 different nucleotide triphosphates and a polymerization agent, under hybridization conditions such that for each nucleotide sequence which has hybridized with a primer, an elongation product of each complementary primer of the matrix is synthesized;
(b) separating the matrix and the elongation product obtained, said elongation product thus also possibly behaving as a matrix;
(c) repeating step a) to obtain a detectable quantity of the desired nucleotide sequences; and
(d) detecting the amplification product of the nucleotide sequences.

## Patentansprüche

1. Protein VanB, **dadurch gekennzeichnet, dass** es die folgende Aminosäurensequenz umfasst, und **dadurch**, dass es bei Gram-positiven Bakterien bei einer Resistenz gegen Vancomycin impliziert ist, wobei diese Resistenz durch Vancomycin induzierbar ist und nicht durch Teicoplanin induzierbar ist.

2. Protein VanB gemäß Anspruch 1, **dadurch gekennzeichnet, dass** es die Fähigkeit aufweist, bei Enterokokken, und zum Beispiel bei Stämmen der Art *E. faecium* oder *E*. *faecalis,* eine Resistenz induzierbarer Art gegen Vancomycin zu verleihen.

3. Protein VanB, **dadurch gekennzeichnet, dass** es eine Aminosäurensequenz umfasst, die bezogen auf die Sequenz wie in Anspruch 1 definiert durch Deletion oder Insertion oder Ersetzen einer oder mehrerer Aminosäuren modifiziert ist, infolgedessen das so modifizierte Protein VanB für das Herstellen einer Resistenz gegen Vancomyin bei Gram-positiven Bakterien erforderlich ist, wobei die Resistenz durch Vancomycin induzierbar ist und nicht durch Teicoplanin induzierbar ist.

4. Peptidfragment des Proteins VanB, **dadurch gekennzeichnet, dass** es der Aminosäurensequenz wie in Anspruch 1 definiert oder jedem Teil dieser Sequenz entspricht, der ausreichend ist, bei Gram-positiven Bakterien, zum Beispiel den Bakterien aus der Familie der Enterokokken, eine Resistenz gegen Vancomycin zu verleihen, wobei die Resistenz durch Vancomycin induzierbar ist und nicht durch Teicoplanin induzierbar ist.

5. Peptidfragment des Proteins VanB, **dadurch gekennzeichnet, dass** es der Aminosäurensequenz wie in Anspruch 1 definiert oder jedem Teil dieser Sequenz entspricht, und **dadurch**, dass es spezifisch von Antikörpern erkannt wird, die sich gegen das Protein VanB richten, und **dadurch**, dass es keine immunologische Reaktion mit Antikörpern, die die Proteine VanA und/oder VanC erkennen, aufweist.

6. Nukleotidsequenz, **dadurch gekennzeichnet, dass** sie für ein Protein VanB kodiert, das bei Gram-positiven Bakterien bei einer Resistenz gegen Vancomycin impliziert ist, wobei die Resistenz durch Vancomycin induzierbar ist und nicht durch Teicoplanin induzierbar ist, wobei das Protein VanB in seinem Peptidskelett die Aminosäurensequenz I, definiert in Anspruch 1, umfasst, oder **dadurch**, dass es sich um eine DNA-Sequenz, die zu dieser kodierenden, Sequenz komplementär ist, oder um eine entsprechende RNA-Sequenz handelt.

7. Nukleotidsequenz gemäß Anspruch 6, **dadurch gekennzeichnet, dass** es sich um die untenstehende Nukleotidkette oder um eine Nukleotidkette handelt, die durch Mutation, Addition oder Deletion von Nukleotiden bezogen auf die untenstehende Nukleotidkette modifiziert ist, infolgedessen sie für ein Protein kodiert, das bei Gram-positiven Bakterien bei einer Resistenz gegen Vancomycin impliziert ist, wobei die Resistenz durch Vancomycin induzierbar ist und nicht durch Teicoplanin induzierbar ist.

8. Nukleotidfragment, **dadurch gekennzeichnet, dass** es in der Lage ist, unter stringenten Bedingungen mit einer Sequenz gemäß einem der Ansprüche 6 oder 7 zu hybridisieren, oder **dadurch**, dass es zu dem hybridisierenden Nukleotidfragment komplementär ist, wobei das Fragment:
(a) ein Fragment von VanB kodiert, definiert gemäß einem der Ansprüche 1 bis 3, das ausreichend ist, um die Resistenz gegen Vancomycin zu verleihen, wobei diese Resistenz durch Vancomycin induzierbar ist und nicht durch Teicoplanin induzierbar ist, oder
(b) die spezifische Detektion von Sequenzen ermöglicht, die das Gen VanB, definiert in Anspruch 6 oder 7, kodieren.

9. Nukleotidfragment gemäß Anspruch 8, **dadurch gekennzeichnet, dass** es unter stringenten Bedingungen nicht mit der DNA von Stämmen von Enterokokken hybridisiert, die gegenüber Vancomycin empfindlich sind, insbesondere mit der DNA der Stämme *E. feacalis* JH2-2, *E. faecium* BM4107.

10. Nukleotidfragment gemäß einem der Ansprüche 8 oder 9, **dadurch gekennzeichnet, dass** seine Sequenz aus der in Anspruch 7 dargestellten Nukleotidkette besteht.

11. Nukleotidfragment gemäß einem der Ansprüche 8 bis 10, **dadurch gekennzeichnet, dass** es markiert ist.

12. Nukleotidfragment gemäß Anspruch 8, **dadurch gekennzeichnet, dass** es sich um eine der folgenden Sequenzen handelt:
- Primer 1: 5' ATGGGAAGCCGATAGTC 3'
- Primer 2: 5' GATTTCGTTCCTCGACC 3'

13. Rekombinante DNA-Sequenz, **dadurch gekennzeichnet, dass** sie eine Nukleotidsequenz gemäß einem der Ansprüche 6 bis 11 umfasst, unter Kontrolle von Regulierungselementen, die geeignet sind, in eine Expression einer Resistenz gegenVancomycin bei einem bestimmten Wirt einzugreifen, wobei die Resistenz durch Vancomycin induzierbar ist und nicht durch Teicoplanin induzierbar ist.

14. Rekombinanter Vektor für das Klonen oder die Expression in einen bestimmten Wirt, **dadurch gekennzeichnet, dass** er eine Nukleotidsequenz gemäß einem der Ansprüche 6 bis 11 umfasst, an einer Stelle, die für seine Replikation nicht wesentlich ist, unter Kontrolle von Regulierungselementen, die geeignet sind, in die Expression einer Resistenz gegen Vancomycin einzugreifen, wobei die Resistenz durch Vancomycin induzierbar ist und nicht durch Teicoplanin induzierbar ist.

15. Rekombinanter Vektor gemäß Anspruch 14, **dadurch gekennzeichnet, dass** es sich um das bei der CNCM unter der Nummer I-1277 hinterlegte Plasmid pAT201 oder um das bei der CNCM unter der Nummer I-1291 hinterlegte Plasmid pAT202 handelt.

16. Rekombinanter Zellwirt, **dadurch gekennzeichnet, dass** er eine Nukleotidsequenz gemäß einem der Ansprüche 6 bis 11 oder einen Vektor gemäß Anspruch 14 oder Anspruch 15 umfasst, wobei dieser Wirt aus den Bakterien, insbesondere den Gram-positiven Kokken, gewählt wird.

17. Monoklonale oder polyklonale Antikörper, **dadurch gekennzeichnet, dass** sie spezifisch das Protein VanB gemäß einem der Ansprüche 1 bis 3 oder ein Peptidfragment gemäß einem der Ansprüche 4 bis 5 erkennen.

18. Kit für die *in* vitro-Diagnose auf einer biologischen Probe der Gegenwart von gegen Vancomycin resistenten Stämmen, wobei diese Stämme zu den Gram-positiven Kokken gehören, insbesondere, dass es sich um Stämme von Enterokokken, zum Beispiel *E. faecium,* handelt, **dadurch gekennzeichnet, dass** es umfasst:
- Antikörper gemäß Anspruch 17, gegebenenfalls markiert,
- ein Reagens für die Detektion einer immunologischen Reaktion der Art Antikörper-Antigen,
- gegebenenfalls Reagenzien, um die Lyse von Zellen der getesteten Probe durchzuführen,
- gegebenenfalls eine bestimmte Vancomycin-Konzentration, um die Resistenz zu induzieren.

19. Kit gemäß Anspruch 18, **dadurch gekennzeichnet, dass** es des Weiteren Antikörper, die sich spezifisch gegen das Protein VanA richten, und/oder Antikörper, die sich spezifisch gegen das Protein VanC richten, umfasst.

20. Kit für die *in* vitro-Diagnose der Gegenwart von gegen Vancomycin resistenten Stämmen, wobei die Stämme insbesondere zu den Gram-positiven Kokken gehören, insbesondere, dass es sich um Stämme von Enterokokken handelt, zum Beispiel *E*. *faecium,* **dadurch gekennzeichnet, dass** es umfasst:
- eine Nukleotidsonde gemäß Anspruch 11, und gegebenenfalls
- Nukleosidtriphosphate dATP, dCTP, dTTP, dGTP,
- ein Mittel zur Polymerisation von DNA.

21. Kit gemäß Anspruch 20, **dadurch gekennzeichnet, dass** es des Weiteren für die Gene VanA und/oder VanC spezifische Nukleotidsonden umfasst.

22. Verfahren zur in vitro-Detektion der Gegenwart von gegen Vancomycin und/oder Teicoplanin resistenten Stämmen, wobei die Stämme zur Familie der Gram-positiven Kokken gehören, insbesondere, dass es sich um Stämme von Enterokokken handelt, zum Beispiel um *E. faecium* oder *E. faecalis,* **dadurch gekennzeichnet, dass** es umfasst:
a) das Inkontaktbringen einer biologischen Probe, die geeignet ist, die resistenten Stämme zu enthalten, mit einem Primer, der aus einem Nukleotidfragment gemäß einem der Ansprüche 8 oder 9 gebildet ist und geeignet ist, mit einer gesuchten Nukleotidsequenz zu hybridisieren, die für die Expression der Resistenz erforderlich ist, wobei diese Sequenz als Matrix verwendet wird, in Gegenwart von 4 unterschiedlichen Nukleosidtriphosphaten und eines Mittel zur Polymerisation, unter derartigen Hybridisierungsbedingungen, dass für jede Nukleotidsequenz, die mit einem Primer hybridisiert wird, ein Produkt zur Verlängerung jedes zu der Matrix komplementären Primers synthetisiert wird,
b) die Trennung der Matrix und des erhaltenen Verlängerungsprodukts, wobei sich das Letztgenannte somit gleichermaßen wie eine Matrix verhalten kann,
c) die Wiederholung des Schritts a), so dass eine detektierbare Menge gesuchter Nukleotidsequenzen erhalten wird,
d) die Detektion des Amplifikationsprodukts der Nukleotidsequenzen.

23. Verfahren zur in vitro-Detektion der Gegenwart von gegen Vancomycin und/oder Teicoplanin resistenten Stämmen, wobei die Stämme insbesondere zur Familie der Gram-positiven Kokken gehören, insbesondere, dass es sich um Stämme von Enterokokken handelt, zum Beispiel um *E. faecium* oder *E. faecalis,* **dadurch gekennzeichnet, dass** es umfasst:
a) das Inkontaktbringen einer biologischen Probe, die geeignet ist, die resistenten Stämme zu enthalten, mit einem Primer, der aus einem Nukleotidfragment gemäß einem der Ansprüche 8 oder 9 gebildet ist, und mit einem Nukleotidfragment des Gens VanA und eines Nukleotidfragments, das für das Gen VanC spezifisch ist und geeignet ist, mit einer gesuchten Nukleotidsequenz zu hybridisieren, die für die Expression der Resistenz erforderlich ist, wobei diese Sequenz als Matrix verwendet wird, in Gegenwart von 4 unterschiedlichen Nukleosidtriphosphaten und eines Mittel zur Polymerisation, unter derartigen Hybridisierungsbedingungen, dass für jede Nukleotidsequenz, die mit einem Primer hybridisiert wird, ein Produkt zur Verlängerung jedes zu der Matrix komplementären Primers synthetisiert wird,
b) die Trennung der Matrix und des erhaltenen Verlängerungsprodukts, wobei sich das Letztgenannte somit gleichermaßen wie eine Matrix verhalten kann,
c) die Wiederholung des Schritts a), so dass eine detektierbare Menge gesuchter Nukleotidsequenzen erhalten wird,
d) die Detektion des Amplifikationsprodukts der Nukleotidsequenzen.


### LISTE DE SEQUENCES

(1) INFORMATION GENERALE:
   (i) DEPOSANT:
      (A) NOM: INSTITUT PASTEUR
      (B) RUE: 25-28, rue du Dr. Roux
      (C) VILLE: PARIS
      (E) PAYS: FRANCE
      (F) CODE POSTAL: 75724 CEDEX 15
   (ii) TITRE DE L' INVENTION: PROTEINE CONFERANT UNE RESISTANCE DE TYPE INDUCTIBLE, A DES GLYCOPEPTIDES, NOTAMMENT CHEZ DES BACTERIES A GRAM-POSITIF. SEQUENCE DE NUCLEOTIDES CODANT POUR CETTE PROTEINE.
   (iii) NOMBRE DE SEQUENCES: 8
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
   (v) DONNEES DE LA DEMANDE ACTUELLE:
      NUMERO DE DEPOT: PCT/FR93/01264
   (vi) DONNEES DE LA DEMANDE ANTERIEURE:
      (A) NUMERO DE DEPOT: FR 9215671
      (B) DATE DE DEPOT: 18-DEC-1992
   (vi) DONNEES DE LA DEMANDE ANTERIEURE:
      (A) NUMERO DE DEPOT: FR 9308356
      (B) DATE DE DEPOT: 07-JUL-1993
(2) INFORMATION POUR LA SEQ ID NO: 1:
   (i) CARACTERISTIQUES DE LA SEQUENCE:
      (A) LONGUEUR: 196 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 1:
   Leu Phe Glu Leu Ser Gly Ile Pro Tyr Val Gly Cys Asp Ile Gln Ser
   1               5                   10                  15
   Ser Ala Ala Cys Met Asp Lys Ser Leu Ala Tyr Ile Leu Thr Lys Asn
               20                  25                  30
   Ala Gly Ile Ala Val Pro Glu Phe Gln Met Ile Glu Lys Gly Asp Lys
           35                  40                  45
   Pro Glu Ala Arg Thr Leu Thr Tyr Pro Val Phe Val Lys Pro Ala Arg
       50                  55                  60
   Ser Gly Ser Ser Phe Gly Val Thr Lys Val Asn Ser Thr Glu Glu Leu
   65                  70                  75                  80
   Asn Ala Ala Ile Glu Ala Ala Gly Gln Tyr Asp Gly Lys Ile Leu Ile
                   85                  90                  95
   Glu Gln Ala Ile Ser Gly Cys Glu Val Gly Cys Ala Val Met Gly Asn
               100                 105                 110
   Glu Asp Asp Leu Ile Val Gly Glu Val Asp Gln Ile Arg Leu Ser His
           115                 120                 125
   Gly Ile Phe Arg Ile His Gln Glu Asn Glu Pro Glu Lys Gly Ser Glu
       130                 135                 140
   Asn Ala Met Ile Ile Val Pro Ala Asp Ile Pro Val Glu Glu Arg Asn
   145                 150                 155                 160
   Arg Val Gln Glu Thr Ala Lys Lys Val Tyr Arg Val Leu Gly Cys Arg
                   165                 170                 175
   Gly Leu Ala Arg Val Asp Leu Phe Leu Gln Glu Asp Gly Gly Ile Val
               180                 185                 190
   Leu Asn Glu Val
           195
(2) INFORMATION POUR LA SEQ ID NO: 2:
   (i) CARACTERISTIQUES DE LA SEQUENCE:
      (A) LONGUEUR: 1140 paires de bases
      (B) TYPE: acide nucléique
      (C) NOMBRE DE BRINS: double
      (D) CONFIGURATION: circulaire
   (ii) TYPE DE MOLECULE: ADN (génomique)
   (ix) CARACTERISTIQUE ADDITIONELLE:
      (A) NOM/CLE: misc_feature
      (B) EMPLACEMENT: 241..258
      (D) AUTRES RENSEIGNEMENTS: /note= "AMORCE 1"
   (ix) CARACTERISTIQUE ADDITIONELLE:
      (A) NOM/CLE: misc_feature
      (B) EMPLACEMENT: 860..877
      (D) AUTRES RENSEIGNEMENTS: /note= "AMORCE 2 : REVERSE COMPLEMENTAIRE"
   (ix) CARACTERISTIQUE ADDITIONELLE:
      (A) NOM/CLE: RBS
      (B) EMPLACEMENT: 52..58
   (ix) CARACTERISTIQUE ADDITIONELLE:
      (A) NOM/CLE: CDS
      (B) EMPLACEMENT: 68..1092
   (xi) DESCRIPTION DE LA SEQUENCE: SEQ ID NO: 2:
   GAGCGTGTGC TGCGAGATAC CACAGAAAAC AATCAGAATT GTCTTAACTT TGAAAGGAGT       60
   TTACAGC ATG AAT AAA ATA AAA GTC GCA ATT ATC TTC GGC GGT TGC TCG        109
           Met Asn Lys Ile Lys Val Ala Ile Ile Phe Gly Gly Cys Ser
             1               5                  10
   GAG GAA CAT GAT GTG TCG GTA AAA TCC GCA ATA GAA ATT GCT GCG AAC        157
   Glu Glu His Asp Val Ser Val Lys Ser Ala Ile Glu Ile Ala Ala Asn
    15                  20                  25                  30
   ATT AAT ACT GAA AAA TTC GAT CCG CAC TAC ATC GGA ATT ACA AAA AAC        205
   Ile Asn Thr Glu Lys Phe Asp Pro His Tyr Ile Gly Ile Thr Lys Asn
                    35                  40                  45
   GGC GTA TGG AAG CTA TGC AAG AAG CCA TGT ACG GAA TGG GAA GCC GAT        253
   Gly Val Trp Lys Leu Cys Lys Lys Pro Cys Thr Glu Trp Glu Ala Asp
                50                  55                  60
   AGT CTC CCC GCC ATA TTC TCC CCG GAT AGG AAA ACG CAT GGT CTG CTT        301
   Ser Leu Pro Ala Ile Phe Ser Pro Asp Arg Lys Thr His Gly Leu Leu
            65                  70                  75
   GTC ATG AAA GAA AGA GAA TAC GAA ACT CGG CGT ATT GAC GTG GCT TTC        349
   Val Met Lys Glu Arg Glu Tyr Glu Thr Arg Arg Ile Asp Val Ala Phe
        80                  85                  90
   CCG GTT TTG CAT GGC AAA TGC GGG GAG GAT GGT GCG ATA CAG GGT CTG        397
   Pro Val Leu His Gly Lys Cys Gly Glu Asp Gly Ala Ile Gln Gly Leu
    95                 100                 105                 110
   TTT GAA TTG TCT GGT ATC CCC TAT GTA GGC TGC GAT ATT CAA AGC TCC        445
   Phe Glu Leu Ser Gly Ile Pro Tyr Val Gly Cys Asp Ile Gln Ser Ser
                   115                 120                 125
   GCA GCT TGC ATG GAC AAA TCA CTG GCC TAC ATT CTT ACA AAA AAT GCG        493
   Ala Ala Cys Met Asp Lys Ser Leu Ala Tyr Ile Leu Thr Lys Asn Ala
               130                 135                 140
   GGC ATC GCC GTC CCC GAA TTT CAA ATG ATT GAA AAA GGT GAC AAA CCG        541
   Gly Ile Ala Val Pro Glu Phe Gln Met Ile Glu Lys Gly Asp Lys Pro
           145                 150                 155
   GAG GCG AGG ACG CTT ACC TAC CCT GTC TTT GTG AAG CCG GCA CGG TCA        589
   Glu Ala Arg Thr Leu Thr Tyr Pro Val Phe Val Lys Pro Ala Arg Ser
       160                 165                 170
   GGT TCG TCC TTT GGC GTA ACC AAA GTA AAC AGT ACG GAA GAA CTA AAC     637
   Gly Ser Ser Phe Gly Val Thr Lys Val Asn Ser Thr Glu Glu Leu Asn
   175                 180                 185                 190
   GCT GCG ATA GAA GCA GCA GGA CAA TAT GAT GGA AAA ATC TTA ATT GAG     685
   Ala Ala Ile Glu Ala Ala Gly Gln Tyr Asp Gly Lys Ile Leu Ile Glu
                   195                 200                 205
   CAA GCG ATT TCG GGC TGT GAG GTC GGC TGC GCG GTC ATG GGA AAC GAG     733
   Gln Ala Ile Ser Gly Cys Glu Val Gly Cys Ala Val Met Gly Asn Glu
               210                 215                 220
   GAT GAT TTG ATT GTC GGC GAA GTG GAT CAA ATC CGG TTG AGC CAC GGT     781
   Asp Asp Leu Ile Val Gly Glu Val Asp Gln Ile Arg Leu Ser His Gly
           225                 230                 235
   ATC TTC CGC ATC CAT CAG GAA AAC GAG CCG GAA AAA GGC TCA GAG AAT     829
   Ile Phe Arg Ile His Gln Glu Asn Glu Pro Glu Lys Gly Ser Glu Asn
       240                 245                 250
   GCG ATG ATT ATC GTT CCA GCA GAC ATT CCG GTC GAG GAA CGA AAT CGG     877
   Ala Met Ile Ile Val Pro Ala Asp Ile Pro Val Glu Glu Arg Asn Arg
   255                 260                 265                 270
   GTG CAA GAA ACG GCA AAG AAA GTA TAT CGG GTG CTT GGA TGC AGA GGG     925
   Val Gln Glu Thr Ala Lys Lys Val Tyr Arg Val Leu Gly Cys Arg Gly
                   275                 280                 285
   CTT GCT CGT GTT GAT CTT TTT TTG CAG GAG GAT GGC GGC ATC GTT CTA     973
   Leu Ala Arg Val Asp Leu Phe Leu Gln Glu Asp Gly Gly Ile Val Leu
               290                 295                 300
   AAC GAG GTC AAT ACC CTG CCC GGT TTT ACA TCG TAC AGC CGC TAT CCA    1021
   Asn Glu Val Asn Thr Leu Pro Gly Phe Thr Ser Tyr Ser Arg Tyr Pro
           305                 310                 315
   CGC ATG GCG GCT GCC GCA GGA ATC ACG CTT CCC GCA CTA ATT GAC AGC    1069
   Arg Met Ala Ala Ala Ala Gly Ile Thr Leu Pro Ala Leu Ile Asp Ser
       320                 325                 330
   CTG ATT ACA TTG GCG ATA GAG AG GTGACCCGTA TGGAAAATGG TTTTTTGTTT    1122
   Leu Ile Thr Leu Ala Ile Glu
   335                 340
   TTTAGATGAA ATGTTGCA                                                1140
(2) INFORMATION POUR LA SEQ ID NO: 3:
   (i) CARACTERISTIQUES DE LA SEQUENCE:
      (A) LONGUEUR: 341 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 3:
   Met Asn Lys Ile Lys Val Ala Ile Ile Phe Gly Gly Cys Ser Glu Glu
    1               5                  10                  15
   His Asp Val Ser Val Lys Ser Ala Ile Glu Ile Ala Ala Asn Ile Asn
               20                  25                  30
   Thr Glu Lys Phe Asp Pro His Tyr Ile Gly Ile Thr Lys Asn Gly Val
           35                  40                  45
   Trp Lys Leu Cys Lys Lys Pro Cys Thr Glu Trp Glu Ala Asp Ser Leu
       50                  55                  60
   Pro Ala Ile Phe Ser Pro Asp Arg Lys Thr His Gly Leu Leu Val Met
   65                  70                  75                  80
   Lys Glu Arg Glu Tyr Glu Thr Arg Arg Ile Asp Val Ala Phe Pro Val
                   85                  90                  95
   Leu His Gly Lys Cys Gly Glu Asp Gly Ala Ile Gln Gly Leu Phe Glu
               100                 105                 110
   Leu Ser Gly Ile Pro Tyr Val Gly Cys Asp Ile Gln Ser Ser Ala Ala
           115                 120                 125
   Cys Met Asp Lys Ser Leu Ala Tyr Ile Leu Thr Lys Asn Ala Gly Ile
       130                 135                 140
   Ala Val Pro Glu Phe Gln Met Ile Glu Lys Gly Asp Lys Pro Glu Ala
   145                 150                 155                 160
   Arg Thr Leu Thr Tyr Pro Val Phe Val Lys Pro Ala Arg Ser Gly Ser
                   165                 170                 175
   Ser Phe Gly Val Thr Lys Val Asn Ser Thr Glu Glu Leu Asn Ala Ala
               180                 185                 190
   Ile Glu Ala Ala Gly Gln Tyr Asp Gly Lys Ile Leu Ile Glu Gln Ala
           195                 200                 205
   Ile Ser Gly Cys Glu Val Gly Cys Ala Val Met Gly Asn Glu Asp Asp
       210                 215                 220
   Leu Ile Val Gly Glu Val Asp Gln Ile Arg Leu Ser His Gly Ile Phe
   225                 230                 235                 240
   Arg Ile His Gln Glu Asn Glu Pro Glu Lys Gly Ser Glu Asn Ala Met
                   245                 250                 255
   Ile Ile Val Pro Ala Asp Ile Pro Val Glu Glu Arg Asn Arg Val Gln
               260                 265                 270
   Glu Thr Ala Lys Lys Val Tyr Arg Val Leu Gly Cys Arg Gly Leu Ala
           275                 280                 285
   Arg Val Asp Leu Phe Leu Gln Glu Asp Gly Gly Ile Val Leu Asn Glu
   290 295 300
   Val Asn Thr Leu Pro Gly Phe Thr Ser Tyr Ser Arg Tyr Pro Arg Met
   305 310 315 320
   Ala Ala Ala Ala Gly Ile Thr Leu Pro Ala Leu Ile Asp Ser Leu Ile
   325 330 335
   Thr Leu Ala Ile Glu
   340
(2) INFORMATION POUR LA SEQ ID NO: 4:
   (i) CARACTERISTIQUES DE LA SEQUENCE:
      (A) LONGUEUR: 589 paires de bases
      (B) TYPE: acide nucléique
      (C) NOMBRE DE BRINS: double
      (D) CONFIGURATION: circulaire
   (ii) TYPE DE MOLECULE: ADN (génomique)
   (xi) DESCRIPTION DE LA SEQUENCE: SEQ ID NO: 4:
   TCTGTTTGAA TTGTCTGGTA TCCCCTATGT AGGCTGCGAT ATTCAAAGCT CCGCAGCTTG    60
   CATGGACAAA TCACTGGCCT ACATTCTTAC AAAAAATGCG GGCATCGCCG TCCCCGAATT   120
   TCAAATGATT GAAAAAGGTG ACAAACCGGA GGCGAGGACG CTTACCTACC CTGTCTTTGT   180
   GAAGCCGGCA CGGTCAGGTT CGTCCTTTGG CGTAACCAAA GTAAACAGTA CGGAAGAACT   240
   AAACGCTGCG ATAGAAGCAG CAGGACAATA TGATGGAAAA ATCTTAATTG AGCAAGCGAT   300
   TTCGGGCTGT GAGGTCGGCT GCGCGGTCAT GGGAAACGAG GATGATTTGA TTGTCGGCGA   360
   AGTGGATCAA ATCCGGTTGA GCCACGGTAT CTTCCGCATC CATCAGGAAA ACGAGCCGGA   420
   AAAAGGCTCA GAGAATGCGA TGATTATCGT TCCAGCAGAC ATTCCGGTCG AGGAACGAAA   480
   TCGGGTGCAA GAAACGGCAA AGAAAGTATA TCGGGTGCTT GGATGCAGAG GGCTTGCTCG   540
   TGTTGATCTT TTTTTGCAGG AGGATGGCGG CATCGTTCTA AACGAGGTC               589
(2) INFORMATION POUR LA SEQ ID NO: 5:
   (i) CARACTERISTIQUES DE LA SEQUENCE:
      (A) LONGUEUR: 17 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (ix) CARACTERISTIQUE ADDITIONELLE:
      (A) NOM/CLE: misc_feature
      (B) EMPLACEMENT: 1..17
      (D) AUTRES RENSEIGNEMENTS: /note= "positions 241-258 de la sequence de nucleotides de SEQ ID NO: 2"
   (xi) DESCRIPTION DE LA SEQUENCE: SEQ ID NO: 5:
      ATGGGAAGCC GATAGTC 17
(2) INFORMATION POUR LA SEQ ID NO: 6:
   (i) CARACTERISTIQUES DE LA SEQUENCE:
      (A) LONGUEUR: 17 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (ix) CARACTERISTIQUE ADDITIONELLE:
      (A) NOM/CLE: misc_feature
      (B) EMPLACEMENT: 1..17
      (D) AUTRES RENSEIGNEMENTS: /note= "Reverse complémentaire du fragment des nucleotides 860 a 877 de SEQ ID NO: 2
   (xi) DESCRIPTION DE LA SEQUENCE: SEQ ID NO: 6:
      GATTTCGTTC CTCGACC 17
(2) INFORMATION POUR LA SEQ ID NO: 7:
   (i) CARACTERISTIQUES DE LA SEQUENCE:
      (A) LONGUEUR: 1079 paires de bases
      (B) TYPE: acide nucléique
      (C) NOMBRE DE BRINS: double
      (D) CONFIGURATION: circulaire
   (ii) TYPE DE MOLECULE: ADN (génomique)
   (ix) CARACTERISTIQUE ADDITIONELLE:
      (A) NOM/CLE: RBS
      (B) EMPLACEMENT: 19..25
   (ix) CARACTERISTIQUE ADDITIONELLE:
      (A) NOM/CLE: CDS
      (B) EMPLACEMENT: 33..1076
   (xi) DESCRIPTION DE LA SEQUENCE: SEQ ID NO: 7:
   AAAGACAGGA AAGAAACTAG GAGGACAAGC AT TTG AAG ATT ATT TTG TTG TAT      53
                                       Leu Lys Ile Ile Leu Leu Tyr
                                        1               5
   GGC GGC AGA AGT GAA GAG CAC GAT GTG TCT GTT TTG TCT GCA TAT TCC     101
   Gly Gly Arg Ser Glu Glu His Asp Val Ser Val Leu Ser Ala Tyr Ser
        10                  15                  20
   GTT TTA AAT GCA ATC TAT TAT AAA TAT TAT CAA GTA CAG TTA GTC TTT     149
   Val Leu Asn Ala Ile Tyr Tyr Lys Tyr Tyr Gln Val Gln Leu Val Phe
    25                  30                  35
   ATT AGT AAA GAC GGT CAA TGG GTA AAA GGC CCT CTT TTA TCT GAA CGA     197
   Ile Ser Lys Asp Gly Gln Trp Val Lys Gly Pro Leu Leu Ser Glu Arg
    40                  45                  50                  55
   CCA CAA AAT AAA GAA GTT TTA CAT TTA ACT TGG GCA CAA ACA CCT GAA     245
   Pro Gln Asn Lys Glu Val Leu His Leu Thr Trp Ala Gln Thr Pro Glu
                    60                  65                  70
   GAA ACA GGC GAA TTT TCA GGA AAA CGA ATC AGT CCT TCG GAA ATT TAT     293
   Glu Thr Gly Glu Phe Ser Gly Lys Arg Ile Ser Pro Ser Glu Ile Tyr
                75                  80                  85
   GAA GAA GAA GCG ATT GTT TTC CCT GTT TTA CAT GGG CCA AAT GGT GAA     341
   Glu Glu Glu Ala Ile Val Phe Pro Val Leu His Gly Pro Asn Gly Glu
            90                  95                 100
   GAT GGA ACA ATT CAA GGA TTC ATG GAA ACC ATT AAT ATG CCT TAT GTA     389
   Asp Gly Thr Ile Gln Gly Phe Met Glu Thr Ile Asn Met Pro Tyr Val
       105                 110                 115
   GGC GCG GGT GTC TTA GCT AGC GTT AAC GCA ATG GAC AAA ATC ATG ACG     437
   Gly Ala Gly Val Leu Ala Ser Val Asn Ala Met Asp Lys Ile Met Thr
   120                 125                 130                 135
   AAA TAT CTT TTA CAA ACT GTT GGC ATT CCA CAA GTA CCA TTC GTG CCA     485
   Lys Tyr Leu Leu Gln Thr Val Gly Ile Pro Gln Val Pro Phe Val Pro
                   140                 145                 150
   GTT TTA AGA AGT GAC TGG AAA GGA AAT CCA AAA GAA GTC TTT GAA AAA     533
   Val Leu Arg Ser Asp Trp Lys Gly Asn Pro Lys Glu Val Phe Glu Lys
               155                 160                 165
   TGT GAA GGT TCT TTA ATT TAT CCG GTC TTT GTT AAA CCT GCC AAT ATG     581
   Cys Glu Gly Ser Leu Ile Tyr Pro Val Phe Val Lys Pro Ala Asn Met
           170                 175                 180
   GGT TCT AGT GTC GGA ATT AGC AAA GTG GAA AAT CGT GAA GAA TTG CAA     629
   Gly Ser Ser Val Gly Ile Ser Lys Val Glu Asn Arg Glu Glu Leu Gln
       185                 190                 195
   GAA GCA TTG GAA GAA GCT TTC CGT TAT GAT GCC CGA GCA ATT GTT GAA     677
   Glu Ala Leu Glu Glu Ala Phe Arg Tyr Asp Ala Arg Ala Ile Val Glu
   200                 205                 210                 215
   CAA GGG ATC GAA GCA CGT GAA ATT GAA GTA GCC ATT TTA GGA AAT GAA         725
   Gln Gly Ile Glu Ala Arg Glu Ile Glu Val Ala Ile Leu Gly Asn Glu
               220                 225                 230
   GAT GTC CGT ACG ACT TTA CCT GGT GAA GTG GTG AAA GAT GTC GCT TTC         773
   Asp Val Arg Thr Thr Leu Pro Gly Glu Val Val Lys Asp Val Ala Phe
               235                 240                 245
   TAT GAT TAT GAT GCA AAA TAC ATC AAT AAC ACG ATT GAA ATG CAA ATC         821
   Tyr Asp Tyr Asp Ala Lys Tyr Ile Asn Asn Thr Ile Glu Met Gln Ile
           250                 255                 260
   CCA GCG CAT GTT CCA GAA GAA GTA GCT CAT CAA GCG CAA GAA TAC GCT         869
   Pro Ala His Val Pro Glu Glu Val Ala His Gln Ala Gln Glu Tyr Ala
       265                 270                 275
   AAA AAA GCG TAT ATT ATG TTA GAT GGA AGT GGC TTA AGT CGC TGT GAT         917
   Lys Lys Ala Tyr Ile Met Leu Asp Gly Ser Gly Leu Ser Arg Cys Asp
   280                 285                 290                 295
   TTC TTC TTA ACA AGC AAA AAC GAA TTA TTC CTG AAT GAA TTG AAC ACC         965
   Phe Phe Leu Thr Ser Lys Asn Glu Leu Phe Leu Asn Glu Leu Asn Thr
               300                 305                 310
   ATG CCT GGT TTT ACT GAC TTT AGT ATG TAT CCT TTA CTG TGG GAA AAT        1013
   Met Pro Gly Phe Thr Asp Phe Ser Met Tyr Pro Leu Leu Trp Glu Asn
               315                 320                 325
   ATG GGC TTG AAA TAC AGT GAT TTA ATT GAG GAA CTG ATT CAG TTA GCT        1061
   Met Gly Leu Lys Tyr Ser Asp Leu Ile Glu Glu Leu Ile Gln Leu Ala
           330                 335                 340
   TTG AAT CGT TTT AAA TAA                                                1079
   Leu Asn Arg Phe Lys
       345
(2) INFORMATION POUR LA SEQ ID NO: 8:
   (i) CARACTERISTIQUES DE LA SEQUENCE:
      (A) LONGUEUR: 348 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 8:
   Leu Lys Ile Ile Leu Leu Tyr Gly Gly Arg Ser Glu Glu His Asp Val
   1               5                   10                  15
   Ser Val Leu Ser Ala Tyr Ser Val Leu Asn Ala Ile Tyr Tyr Lys Tyr
               20                  25                  30
   Tyr Gln Val Gln Leu Val Phe Ile Ser Lys Asp Gly Gln Trp Val Lys
           35                  40                  45
   Gly Pro Leu Leu Ser Glu Arg Pro Gln Asn Lys Glu Val Leu His Leu
        50                  55                  60
   Thr Trp Ala Gln Thr Pro Glu Glu Thr Gly Glu Phe Ser Gly Lys Arg
    65                  70                  75                  80
   Ile Ser Pro Ser Glu Ile Tyr Glu Glu Glu Ala Ile Val Phe Pro Val
                    85                  90                  95
   Leu His Gly Pro Asn Gly Glu Asp Gly Thr Ile Gln Gly Phe Met Glu
               100                 105                 110
   Thr Ile Asn Met Pro Tyr Val Gly Ala Gly Val Leu Ala Ser Val Asn
           115                 120                 125
   Ala Met Asp Lys Ile Met Thr Lys Tyr Leu Leu Gln Thr Val Gly Ile
       130                 135                 140
   Pro Gln Val Pro Phe Val Pro Val Leu Arg Ser Asp Trp Lys Gly Asn
   145                 150                 155                 160
   Pro Lys Glu Val Phe Glu Lys Cys Glu Gly Ser Leu Ile Tyr Pro Val
                   165                 170                 175
   Phe Val Lys Pro Ala Asn Met Gly Ser Ser Val Gly Ile Ser Lys Val
               180                 185                 190
   Glu Asn Arg Glu Glu Leu Gln Glu Ala Leu Glu Glu Ala Phe Arg Tyr
           195                 200                 205
   Asp Ala Arg Ala Ile Val Glu Gln Gly Ile Glu Ala Arg Glu Ile Glu
       210                 215                 220
   Val Ala Ile Leu Gly Asn Glu Asp Val Arg Thr Thr Leu Pro Gly Glu
   225                 230                 235                 240
   Val Val Lys Asp Val Ala Phe Tyr Asp Tyr Asp Ala Lys Tyr Ile Asn
                   245                 250                 255
   Asn Thr Ile Glu Met Gln Ile Pro Ala His Val Pro Glu Glu Val Ala
               260                 265                 270
   His Gln Ala Gln Glu Tyr Ala Lys Lys Ala Tyr Ile Met Leu Asp Gly
           275                 280                 285
   Ser Gly Leu Ser Arg Cys Asp Phe Phe Leu Thr Ser Lys Asn Glu Leu
       290                 295                 300
   Phe Leu Asn Glu Leu Asn Thr Met Pro Gly Phe Thr Asp Phe Ser Met
   305                 310                 315                 320
   Tyr Pro Leu Leu Trp Glu Asn Met Gly Leu Lys Tyr Ser Asp Leu Ile
                   325                 330                 335
   Glu Glu Leu Ile Gln Leu Ala Leu Asn Arg Phe Lys
               340                 345